(19) Europäisches Patentamt
European Patent Office
Office européen des brevets

(11) **EP 2 599 768 A1**

(12) **EUROPEAN PATENT APPLICATION**
published in accordance with Art. 153(4) EPC


(43) Date of publication:
**05.06.2013 Bulletin 2013/23**

(21) Application number: **11812062.5**

(22) Date of filing: **27.07.2011**

(51) Int Cl.:
***C07C 211/61*** *(2006.01)* ***H01L 51/42*** *(2006.01)*

(86) International application number:
**PCT/JP2011/004232**

(87) International publication number:
**WO 2012/014460 (02.02.2012 Gazette 2012/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.07.2010 JP 2010168355**
**27.07.2010 JP 2010168354**



(71) Applicant: **Idemitsu Kosan Co., Ltd.**
**Chiyoda-ku**
**Tokyo 100-8321 (JP)**

(72) Inventors:
- **YASUKAWA, Keiichi**
  **Sodegaura-shi**
  **Chiba 299-0293 (JP)**
- **IWAMOTO, Shintaro**
  **Sodegaura-shi**
  **Chiba 299-0293 (JP)**
- **IKEDA, Hidetsugu**
  **Sodegaura-shi**
  **Chiba 299-0293 (JP)**
- **TOKAILIN, Hiroshi**
  **Sodegaura-shi**
  **Chiba 299-0293 (JP)**

(74) Representative: **Gille Hrabal**
**Patentanwälte**
**Brucknerstrasse 20**
**40593 Düsseldorf (DE)**


(54) **INDENOPERYLENE COMPOUND, MATERIAL FOR ORGANIC THIN-FILM PHOTOVOTAIC CELL CONTAINING INDENOPERYLENE DERIVATIVE, AND ORGANIC THIN-FILM PHOTOVOTAIC CELL USING SAME**


(57) An indenoperylene derivative represented by a formula (A-1), wherein in the formula (A-1), at least one of $R_1$ to $R_{16}$ is an amino group represented by the formula (A-2). In the formula (A-2), $R_a$ and $R_b$ are a substituted or unsubstituted aryl group having 6 to 40 ring carbon atoms or a substituted or unsubstituted alkyl group having 1 to 40 carbon atoms.

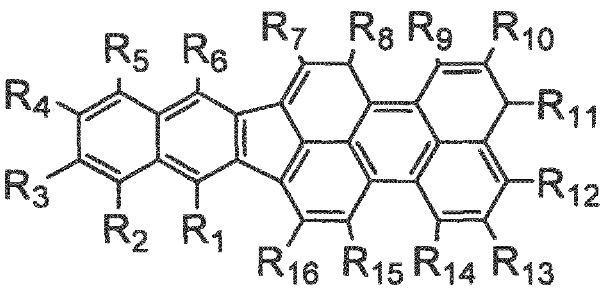

(A-1)

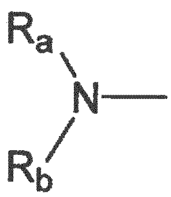

(A-2)


EP 2 599 768 A1

## Description

Technical Field

The invention relates to a novel indenoperylene derivative, a material for an organic thin film solar cell comprising the same, and an organic thin film solar cell using the same. The invention relates to a material for an organic thin film solar cell comprising an indenoperylene derivative and an organic thin film solar cell using the same.

Background Art

An organic thin film solar cell is a device which outputs electric power through input of light In this regard, it is a device which shows a response opposite to an organic electroluminescence (EL) device which outputs light through input of electric power. A solar cell including an organic thin film solar cell has attracted remarkable attention in recent years as a source of clean energy backed by the problem of running out of fossil fuels or global warming.
Heretofore, solar cells which have been put into practical use are silicon-based ones using monocrystalline Si, polycrystalline Si, amorphous Si, or the like. However, there is an increasing demand for a next-generation solar cell since the silicon-based solar cell is expensive and the shortage of Si as the raw material and other problems have come up to the surface. Under such circumstances, an organic solar cell has attracted attention as a next-generation solar cell replacing a silicon-based solar cell since it is inexpensive and is free of fear of shortage of raw materials.

An organic solar cell is basically composed of an n layer which transfers electrons and a p layer which transfers holes.
A solar cell in which, a layer obtained by subjecting a sensitizing dye such as a ruthenium dye to monomolecular adsorption on the surface of an inorganic semiconductor such as titania is used as the n layer, and an electrolyte solution is used as the p layer, is called as a dye-sensitized solar cell (so-called a Graetzel cell). Researches on the dye-sensitized solar cell have been energetically conducted since 1991 in view of its high photoelectric conversion efficiency (hereinafter often abbreviated as "conversion efficiency"). However, it has defects that leakage occurs after the use for a long period of time due to the use of a solution, or the like.
In order to overcome such defects, researches to obtain an all solid-type dye-sensitized solar cell by solidifying an electrolyte solution have been made recently. However, the technology of allowing an organic substance to be perfused to fine pores of porous titania is very difficult, and therefore, a cell which exhibits a high conversion efficiency with a good reproducibility has not yet been completed.

On the other hand, an organic thin film solar cell in which both of the n layer and the p layer are formed from organic thin films has no defect such as leakage of the solution because it is an all solid-type cell. In addition, it can be easily fabricated, uses no ruthenium which is a rare metal, and has other advantages. Therefore, such an organic thin film solar cell has attracted attention and has been energetically studied.
As for organic thin film solar cells, studies have been made in the early stage on a monolayer film formed of a merocyanine dye or the like. It was found that the conversion efficiency increases by using a multilayer film of a p layer/n layer, and thereafter, such a multilayer film has been mainly employed. The materials used at that time were copper phthalocyanine (CuPc) for the p layer and peryleneimides (PTCBI) for the n layer.
Thereafter, it was found that inserting an i layer (a mixture layer of a p-material and an n-material) between the p layer and the n layer results in improvement in the conversion efficiency. However, the materials used at that time were phthalocyanines and peryleneimides as ever.
Subsequently, it was found that the conversion efficiency is further improved by employing a stack cell structure in which several p/i/n layers are stacked. The materials used at that time were phthalocyanines and fullerene $C_{60}$.

In an organic thin film solar cell using a polymer compound, studies have been mainly made on the so-called bulkhetero structure in which a conductive polymer is used as the p material (a material used in the p layer) and a $C_{60}$ derivative is used as the n material (a material used in the n layer). These materials are mixed and the resulting mixture is subjected to a heat treatment to induce the separation of micro layers and allow the hetero interface to grow, thereby to improve the conversion efficiency. The materials used mainly in this structure were a soluble polythiophene derivative called as P3HT (poly-3-hexylthiophene) as the p material and a soluble $C_{60}$ derivative called as PCBM (phenyl-C61-butyric acid methyl ester) as the n material.

As mentioned above, in an organic thin film solar cell, the conversion efficiency was improved by optimizing the cell structure and the morphology of the p material and the n material. However, no significant change was made for the materials used in an organic thin film solar cell, and phthalocyanine-based materials, peryleneimide-based materials and $C_{60}$ derivative-based materials have still been used. Therefore development of new materials replacing these materials has been aspired.

In general, the operation process of an organic solar cell is essentially composed of (1) absorption of light and generation of excitons, (2) diffusion of excitons, (3) separation of electric charges, (4) movement of carriers and (5)

generation of an electromotive force. In general, not many organic substances show absorption properties which coincident with the solar light spectrum, and hence, an organic thin film solar cell could not attain a high conversion efficiency.

In order to improve performance in any of the above-mentioned processes to improve conversion efficiency, studies have been made on development of a new material.

For example, Patent Document 1 discloses an organic photoelectric conversion device utilizing a periflanthene derivative. However, due to its basic structure having a benzofluoranthene skeleton on the both sides of the perylene skeleton, a periflanthene derivative has a large molecular weight. Therefore, when an attempt is made to produce an organic thin film solar cell using the vapor deposition method, problems relating to the production such as difficulty in sublimation may occur.

Patent Document 2 discloses an organic thin film solar cell utilizing a compound in which an aromatic amine portion is introduced into an acenaphthofluoranthene skeleton is disclosed. Although a high conversion efficiency is disclosed in the examples, a further increase in efficiency has been demanded.

In addition to the compounds mentioned above, an arylamine compound has been found as the hole-transporting material and the hole-injecting material during the course of development of an organic EL device. The arylamine compound has excellent hole-transporting performance, and hence, its use as the p material of an organic thin film solar cell was considered. However, an arylamine compound does not show light absorption in the visible range and has an insufficient absorption property for the spectrum of the sun, and hence, the photoelectric conversion efficiency was not sufficient. This demonstrates difficulty in using an organic EL material to an organic thin film solar cell.

In general, in an organic compound, it is known that the $\pi$-electron conjugated system is expanded to allow the absorption maximum wavelength to be shifted to a long wavelength side in order to allow an organic compound to have an absorption in the visible light range. However, if the molecular weight becomes too large by expanding the conjugated system too much, problems that solubility in a solvent is lowered to lead to difficulty in purification, purification by sublimation becomes impossible due to an increase in sublimation temperature or the like have come up to the surface. Therefore, as one example of the technique to shift the absorption wavelength to a long wavelength side efficiently while suppressing the molecular weight to some degree, use of a polyacene is known.

Patent Documents 3 and 4 disclose a material for a solar cell in which a polyacene compound is used. However, in general, if the number of fused rings is increased in order to expand the visible light absorption range, a polyacene becomes instable to light or oxygen, and as a result, purification or handling becomes difficult, and the purity thereof is hard to be increased. Therefore, an organic solar cell using polyacene cannot be said as a practical solar cell material.

Patent Document 5 discloses an organic EL device utilizing an indenoperylene compound. However, no disclosure or suggestion is made on its application in organic thin film solar cells.

Patent Document 1 discloses an organic photoconversion device using a perylene derivative in its electron donor layer. However, a perylene derivative having a diarylamino group and/or a dialkylamino group is not disclosed. Further, Patent Document 6 discloses an indenopyrene compound having an arylamine substituent and an organic thin film solar cell. However, for the organic thin film solar cell, a further higher conversion efficiency has been required.

Related Art Documents

Patent Documents

Patent Document 1: JP-A-2008-135540
Patent Document 2: JP-A-2009-132674
Patent Document 3: JP-A-2008-34764
Patent Document 4: JP-A-2007-335760
Patent Document 5: WO2001/023497
Patent Document 6: WO2010/01352

Summary of the Invention

An object of the invention is to provide an indenoperylene derivative useful as a material for an organic thin film solar cell. In particular, an object of the invention is to provide a compound exhibiting a highly degree of photoelectric conversion efficiency when used in an organic thin film solar cell.

An object of the invention is to provide a material for an organic thin film solar cell capable of exhibiting excellent photoelectric conversion efficiency when used in an organic thin film solar cell.

According to the invention, the following indenoperylene derivative or the like are provided.

1. An indenoperylene derivative represented by a formula (A-1):

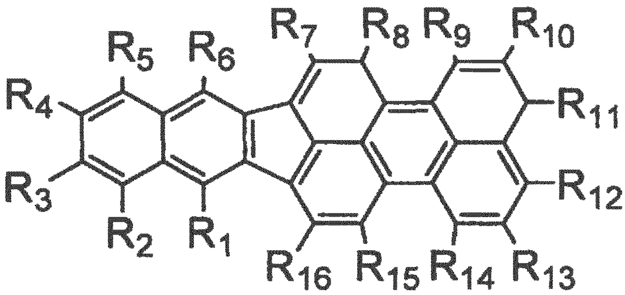

(A-1)

wherein in the formula (A-1), $R_1$ to $R_{16}$ are independently a hydrogen atom, a substituted or unsubstituted alkyl group having 1 to 40 carbon atoms, a substituted or unsubstituted alkenyl group having 2 to 40 carbon atoms, a substituted or unsubstituted alkynyl group having 2 to 40 carbon atoms, a substituted or unsubstituted aryl group having 6 to 40 ring carbon atoms, a substituted or unsubstituted heteroaryl group having 5 to 40 ring atoms, a substituted or unsubstituted alkoxy group having 1 to 40 carbon atoms, a substituted or unsubstituted aryloxy group having 6 to 40 ring carbon atoms or an amino group represented by the formula (A-2), and at least one of $R_1$ to $R_{16}$ is an amino group represented by the formula (A-2):

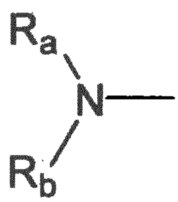

(A-2)

wherein $R_a$ and $R_b$ are independently a substituted or unsubstituted aryl group having 6 to 40 ring carbon atoms or a substituted or unsubstituted alkyl group having 1 to 40 carbon atoms.

2. The indenoperylene derivative according to 1, which is represented by a formula (A-3):

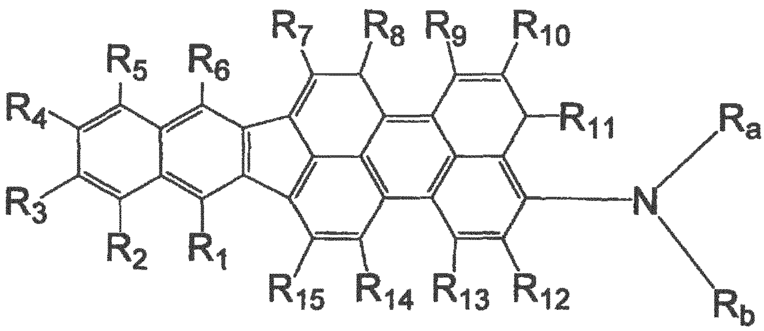

(A-3)

wherein in the formula (3), $R_1$ to $R_{15}$ and $R_a$ and $R_b$ are the same as those in the formulas (A-1) and (A-2).

3. The indenoperylene derivative according to 1 or 2, which is represented by a formula (A-4):

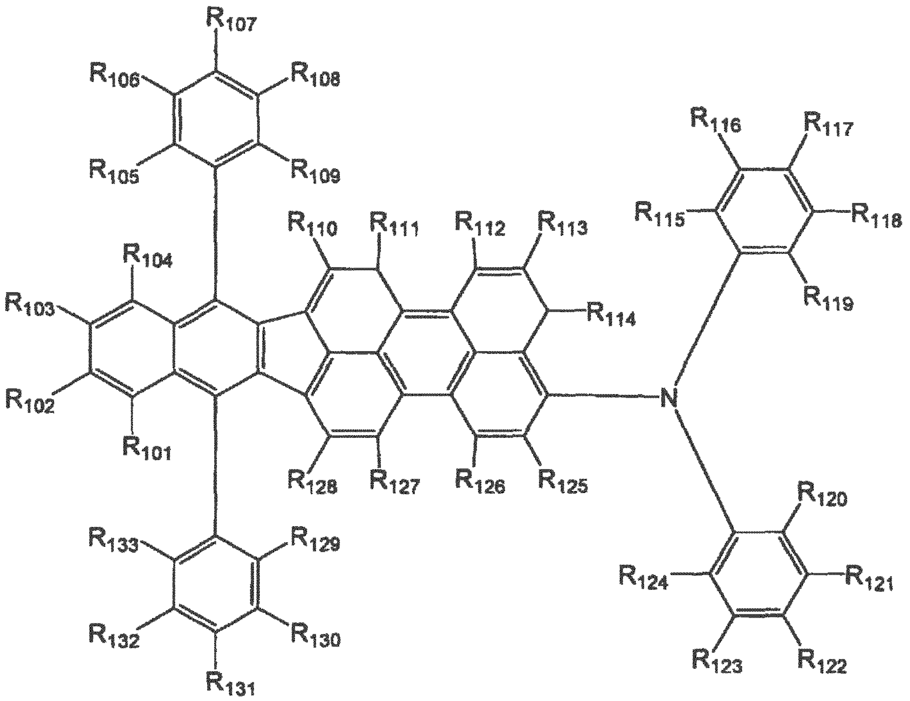

(A-4)

wherein $R_{101}$ to $R_{133}$ are independently a hydrogen atom, a substituted or unsubstituted alkyl group having 1 to 40 carbon atoms, a substituted or unsubstituted aryl group having 6 to 40 ring carbon atoms, a substituted or unsubstituted heteroaryl group having 5 to 40 ring atoms or a substituted or unsubstituted alkoxy group having 1 to 40 carbon atoms.

4. A material for an organic thin film solar cell comprising the indenoperylene derivative according to any of 1 to 3.

5. An organic thin film solar cell comprising the indenoperylene derivative according to any of 1 to 3.

6. An organic thin film solar cell comprising at least a p layer, wherein the p layer comprises the indenoperylene derivative according to any of 1 to 3.

7. An organic thin film solar cell comprising at least a p layer and an n layer, wherein the n layer comprise a fullerene derivative and the p layer comprises the indenoperylene derivative according to any of 1 to 3.

8. A material for an organic thin film solar cell comprising an indenoperylene derivative represented by the following formula (B-1):

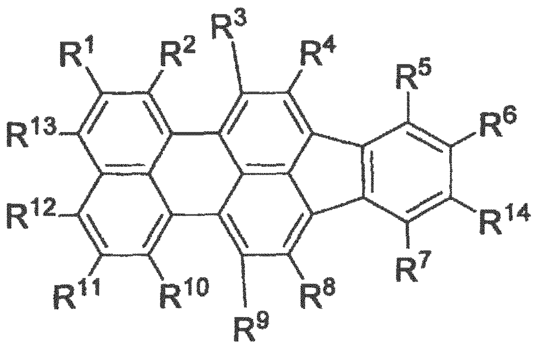

(B-1)

wherein $R^1$ to $R^{14}$ are independently an amino group represented by the following formula (B-2), a hydrogen atom, a halogen atom, a substituted or unsubstituted alkyl group having 1 to 40 carbon atoms, a substituted or unsubstituted alkenyl group having 2 to 40 carbon atoms, a substituted or unsubstituted alkynyl group having 2 to 40 carbon atoms, a substituted or unsubstituted aryl group having 6 to 40 ring carbon atoms, a substituted or unsubstituted heteroaryl group having 5 to 40 ring atoms, a substituted or unsubstituted alkoxy group having 1 to 40 carbon atoms or a substituted or unsubstituted aryloxy group having 6 to 40 ring carbon atoms, at least one of $R^1$ to $R^{14}$ is an amino group represented by the following formula (B-2); and
adjacent groups of $R^1$ to $R^{14}$ may be bonded to each other to form a ring:

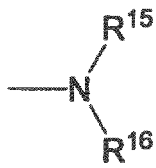

(B-2)

wherein $R^{15}$ and $R^{16}$ are independently a substituted or unsubstituted aryl group having 6 to 40 ring carbon atoms or a substituted or unsubstituted alkyl group having 1 to 40 carbon atoms.

9. The material for an organic thin film solar cell according to 8, wherein the indenoperylene derivative represented by the formula (B-1) is represented by the following formula (B-3):

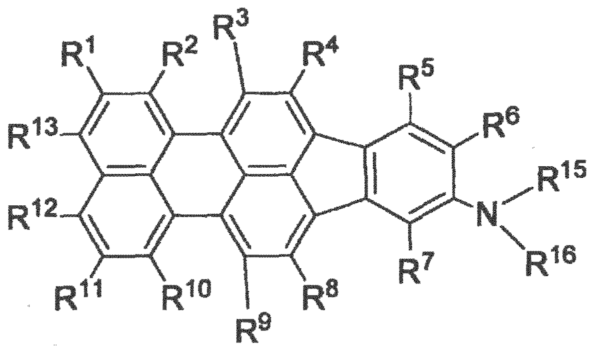

(B-3)

wherein $R^1$ to $R^{13}$ and $R^{15}$ and $R^{18}$ are the same as those in the formulas (B-1) and (B-2).

10. An organic thin film solar cell comprising the material for an organic thin film solar cell according to 8 or 9.

11. An organic thin film solar cell having at least a p layer, wherein the p layer comprises the material for an organic thin film solar cell according to 8 or 9.

12. An organic thin film solar cell comprising at least a p layer and an n layer, wherein the n layer comprises a fullerene derivative and the p layer comprises the material for an organic thin film solar cell according to 8 or 9.

According to the invention, an indenoperylene derivative which is useful as a material of an organic thin film solar cell can be provided. In particular, a compound having a high photoelectric conversion efficiency when used in an organic thin film solar cell can be provided.
According to the invention, a material for an organic thin film solar cell which exhibits excellent photoelectric conversion performance when used in an organic thin film solar cell can be provided.

Mode for Carrying out the Invention

The first aspect of the invention will be explained.
The indenoperylene derivative according to the first aspect of the invention (hereinafter often simply referred to as the "first indenoperylene derivative") is represented by the formula (A-1):

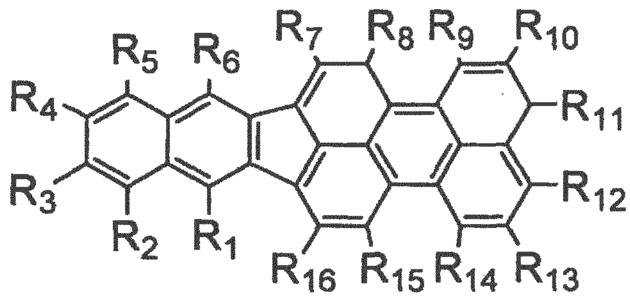

(A-1)

in the formula (A-1), $R_1$ to $R_{16}$ independently are a hydrogen atom, a substituted or unsubstituted alkyl group having 1 to 40 carbon atoms, a substituted or unsubstituted alkenyl group having 2 to 40 carbon atoms, a substituted or unsubstituted alkynyl group having 2 to 40 carbon atoms, a substituted or unsubstituted aryl group having 6 to 40 carbon atoms

that form a ring (hereinafter referred to as the "ring carbon atoms"), a substituted or unsubstituted heteroaryl group having 5 to 40 atoms that form a ring (hereinafter referred to as the "ring atom"), a substituted or unsubstituted alkoxy group having 1 to 40 carbon atoms, a substituted or unsubstituted aryloxy group having 6 to 40 ring carbon atoms or an amino group represented by the formula (A-2), and at least one of $R_1$ to $R_{16}$ is an amino group represented by the formula (A-2).

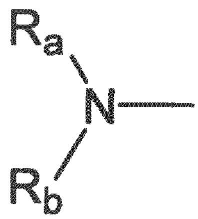
(A-2)

In the formula (A-2), $R_a$ and $R_b$ are independently a substituted or unsubstituted aryl group having 6 to 40 ring carbon atoms or a substituted or unsubstituted alkyl group having 1 to 40 carbon atoms.

The compound represented by the formula (A-1) has an indenoperylene structure in which it has a benzofluoranthene skeleton on only one side of the perylene skeleton, whereby an increase in molecular weight is suppressed. When this compound is used as a p material of an organic thin film solar cell, a high conversion efficiency is obtained. When an organic thin film solar cell is produced by the vapor deposition method, since sublimation is easy, lowering in productivity due to difficulty in sublimation can be eliminated.

The reason that the structure of (A-2) is essential in the compound represented by the formula (A-1) is as follows. By introducing an amino group having excessive electrons (such as a diarylamino group or a dialkylamino group, or the like), transporting properties of holes are increased, whereby conversion efficiency is improved when used as an element of an organic thin film solar cell.

In the formula (A-1), in respect of easiness in production of a compound, it is preferred that one or more of $R_3$, $R_4$, $R_{11}$ and $R_{12}$ be an amino group represented by the formula (A-2), and $R_1$, $R_2$, $R_5$ to $R_{10}$, and $R_{13}$ to $R_{16}$ be a group other than that represented by the formula (A-2) or a hydrogen atom.
Further, when the compound represented by the formula (A-1) is used as an element of an organic thin film solar cell, it is preferred that the number of the amino groups in the formula (A-2) be 1 since electron charges are stably transferred. Therefore, the compounds represented by the formulas (A-3) and (A-4) are preferable.

$R_a$ and $R_b$ in the formula (A-2) is a substituted or unsubstituted aryl group having 6 to 40 ring carbon atoms or a substituted or unsubstituted alkyl group having 1 to 40 carbon atoms. Specific examples thereof are the same as those for an aryl group or an alkyl group represented by the following $R_1$ to $R_{16}$.
Due to the stable presence of a radical cation (hole), stable transfer of electric charges can be obtained. In this respect, an aryl group is preferable. As the aryl group, a phenyl group, a naphthyl group, a biphenyl group or the like are preferable.

The first indenoperylene derivative of the invention is preferably represented by the formula (A-3):

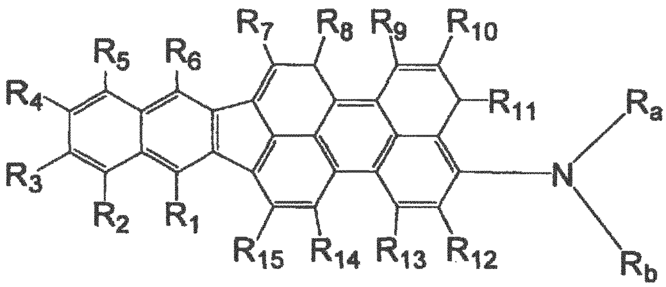
(A-3)

In the formula (A-3), $R_1$ to $R_{15}$ and $R_a$ and $R_b$ are the same as those in the formulas (A-1) and (A-2).

The compound represented by the formula (A-3) is a compound in which $R_{11}$ or $R_{12}$ in the formula (A-1) is represented by the formula (A-2). Since the $R_{11}$ and $R_{12}$ in the formula (A-1) are the sites to which an amino group tends to be introduced easily, in respect of productivity, as the compound having one amino group, a compound represented by the formula (A-3) is preferable.

The first indenoperylene derivative of the invention is preferably represented by the formula (A-4).
A structure in which $R_1$, $R_6$, $R_a$ and $R_b$ in the formula (A-3) are a phenyl group which may have a substituent is represented by the formula (A-4). This structure is similar to mTPD (hole-transporting material of an organic EL device) due to the

presence of a diphenylamino group or four phenyl groups. Therefore, it is considered that a further high charge transfer can be obtained and that a high conversion efficiency shown in the Examples can be obtained.

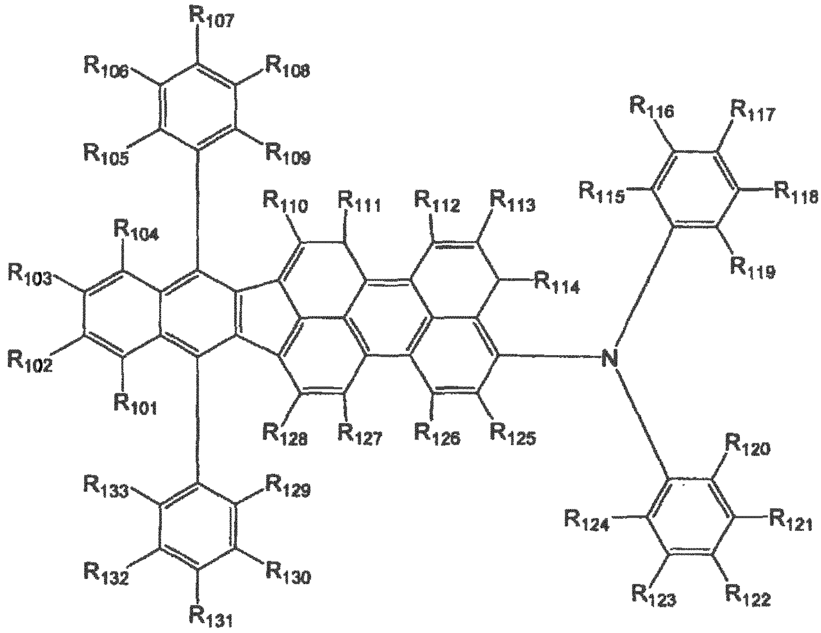

(A−4)

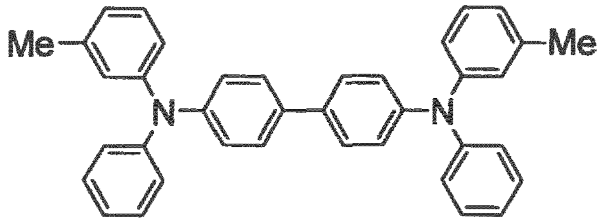

mTPD

in the formula, $R_{101}$ to $R_{133}$ are independently a hydrogen atom, a substituted or unsubstituted alkyl group having 1 to 40 carbon atoms, a substituted or unsubstituted aryl group having 6 to 40 ring carbon atoms, a substituted or unsubstituted heteroaryl group having 5 to 40 ring atoms or a substituted or unsubstituted alkoxy group having 1 to 40 carbon atoms. Specific examples thereof are the same as those for the following $R_1$ to $R_{16}$.

The four phenyl groups represented by the formula (A-4) may have a substituent. However, an unsubstituted phenyl group is preferable. However, there may be a case where a bulky alkyl substituent such as a t-butyl group may contribute to an increase in conversion efficiency.

Hereinbelow, an explanation will be made on each substituent of the first indenoperylene derivative.

The substituted or unsubstituted alkyl group having 1 to 40 carbon atoms represented by $R_1$ to $R_{16}$ may be liner, branched or cyclic. Specific examples thereof include a methyl group, an ethyl group, a 1-propyl group, a 2-propyl group, a 1-butyl group, a 2-butyl group, a sec-butyl group, a tert-butyl group, various pentyl groups, various hexyl groups, various octyl groups, various decyl groups, various dodecyl groups, 2-ethylhexyl group, a 3,7-dimethylocyl group, a cyclopropyl group, a cyclopentyl group, a cyclohexyl group, a 1-adamantyl group, 2-adamantyl group, a norbornyl group, a trifluoromethyl group, a trichloromethyl group, a benzyl group, an $\alpha,\alpha$-dimethylbenzyl group, a 2-phenylethyl group, and a 1-phenylethyl group or the like. Of these, in respect of availability of a raw material or for other reasons, a methyl group, an ethyl group, a 1-propyl group, a 2-propyl group, a tert-butyl group and a cyclohexyl group are preferable.

As the substituent of the above-mentioned alkyl group, a halogen atom such as a fluorine atom, a chlorine atom, a bromine atom and an iodine atom, and an aryl group such as a phenyl group can be given. Further, as the substituent which further connects to the aryl group, an alkyl group having 1 to 5 carbon atoms such as a methyl group, an ethyl group and a propyl group can be given.

The substituted or unsubstituted alkenyl group having 2 to 40 carbon atoms represented by $R_1$ to $R_{16}$ may be

linear, branched or cyclic. Specific examples thereof include a vinyl group, a propenyl group, a butenyl group, an oleyl group, an eicosapentaenyl group, a docosahexaenyl group, a styryl group, a 2,2-diphenylvinyl group, a 1,2,2-triphenylvinyl group, a 2-phenyl-2-propenyl group or the like can be given. Of these, in respect of easiness in obtaining a raw material or the like, a vinyl group, a styryl group, a 2,2-diphenylvinyl group are preferable.
As the substituent for the alkenyl group, a halogen atom such as a fluorine atom, a chlorine atom, a bromine atom and an iodine atom, and an aryl group such as a phenyl group can be given.

The substituted or unsubstituted alkynyl group having 2 to 40 carbon atoms represented by $R_1$ to $R_{16}$ may be linear, branched or cyclic. Specific examples thereof include an ethenyl group, a propynyl group, a 2-phenylethenyl group or the like.
As the substituent of the alkynyl group, a halogen atom such as a fluorine atom, a chlorine atom, a bromine atom and an iodine atom and an aryl group such as a phenyl group can be given.

Specific examples of the substituted or unsubstituted aryl group having 6 to 40 ring carbon atoms represented by $R_1$ to $R_{16}$ include a phenyl group, a 2-tolyl group, a 4-tolyl group, a 4-trifluoromethylphenyl group, a 4-methoxyphenyl group, a 4-cyanophenyl group, a 2-biphenylyl group, a 3-biphenylyl group, a 4-biphenylyl group, a terphenylyl group, a 3,5-diphenylphenyl group, a 3,4-diphenylphenyl group, a pentaphenyphenyl group, a 4-(2,2-diphenylvinyl)phenyl group, a 4-(1,2,2-triphenylvinyl)phenyl group, a fluorenyl group, a 1-naphthyl group, a 2-naphthyl group, a 9-anthryl group, a 2-anthryl group, a 9-phenanthryl group, a 1-pyrenyl group, a chrysenyl group, a naphthacenyl group, and a coronenyl group. Of these, a phenyl group, a 4-biphenyl group, a 1-naphthyl group, a 2-naphthyl group, a 9-phenanthryl group or the like are preferable in respect of easiness in obtaining raw materials or for other reasons.
As the substituent for the above-mentioned aryl group, a halogen atom such as a fluorine atom, a chlorine atom, a bromine atom and an iodine atom, an alkoxy group having 1 to 5 carbon atoms such as a methoxy group, an ethoxy group and a propoxy group, a cyano group and a vinyl group substituted by an aryl group such as a phenyl group can be given.

As for the substituted or unsubstituted heteroaryl group having 5 to 40 ring atoms represented by $R_1$ to $R_{16}$, for example, furan, thiophene, pyrrole, imidazole, benzimidazole, pyrazole, benzpyrazole, triazole, oxadiazole, pyridine, pyrazine, triazine, quinoline, benzofuran, dibenzofuran, benzothiophene, dibenzothiophene or a heteroaryl group based on carbazole can be given. Of these, furan, thiophene, pyridine, a heteroaryl group based on carbazole or the like are preferable in respect of easiness in obtaining raw materials or for other reasons.
If the heteroaryl group is based on the nitrogen-containing azole-based heterocyclic ring, the bonding position of the indenoperylene skeleton represented by the formula (A-1) may be carbon or nitrogen.
As the substituent of the above-mentioned heteroaryl group, a halogen atom such as a fluorine atom, a chlorine atom, a bromine atom and an iodine atom, an alkoxy group such as a methoxy group, an ethoxy group and a propoxy group, a cyano group and a vinyl group substituted by an aryl group such as a phenyl group can be given.

The substituted or unsubstituted alkoxy group having 1 to 40 carbon atoms represented by $R_1$ to $R_{16}$ may be linear, branched or cyclic. Specific examples thereof include a methoxy group, an ethoxy group, a 1-propyloxy group, a 2-propyloxy group, a 1-butyloxy group, a 2-butyloxy group, a sec-butyloxy group, a tert-butyloxy group, a various pentyloxy groups, various hexyloxy groups, various octyloxy groups, various decyloxy groups, various dodecyloxy groups, a 2-ethylhexyloxy group, a 3,7-dimethyloctyloxy group, a cyclopropyloxy group, a cyclopentyloxy group, a cyclohexyloxy group, a 1-adamantyloxy group, a 2-adamantyloxy group, a norbomyloxy group, a trifluoromethoxy group, a benzyloxy group, an a,a-dimethylbenryloxy group, a 2-phenylethoxy group and a 1-phenylethoxy group. Of these, a methoxy group, an ethoxy group, a tert-butyloxy group and the like are preferable in respect of easiness in obtaining raw materials or for other reasons.
As the substituent for the above-mentioned alkoxy group, for example, a halogen atom such as a fluorine atom, a chlorine atom, a bromine atom and an iodine atom; and an aryl group such as a phenyl group, and as a substituent which is further connected to the aryl group, an alkyl group having 1 to 5 carbon atoms such as a methyl group, an ethyl group and a propyl group can be given.

Specific examples of the substituted or unsubstituted aryloxy group having 6 to 40 ring carbon atoms represented by $R_1$ to $R_{16}$ include a substituent in which the above-mentioned aryl groups are bonded through oxygen. Of these, a phenoxy group, a naphthoxy group, a phenanthryloxy group or the like are preferable in respect of easiness in obtaining raw materials or for other reasons.
As the substituent for the above-mentioned aryloxy group, for example, a halogen atom such as a fluorine atom, a chlorine atom, a bromine atom and an iodine atom; and an alkoxy group having 1 to 5 carbon atoms such as a methoxy group, an ethoxy group and a propoxy group, a cyano group, and a vinyl group substituted by an aryl group such as a phenyl group can be given.

As the first indenoperylene derivative of the invention, the following compounds can be given, for example.

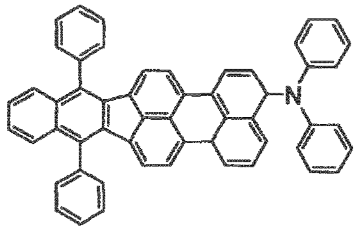

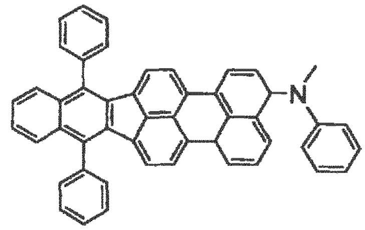

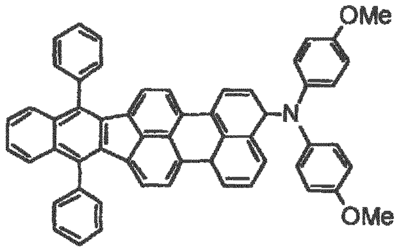

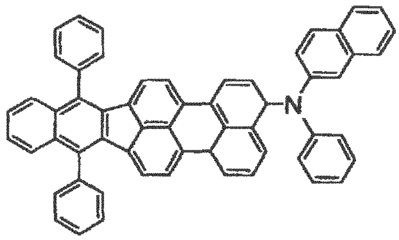

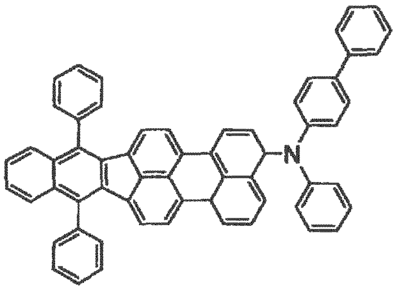

Hereinbelow, the second aspect of the invention will be explained.

The material for the organic thin film solar cell according to the second aspect of the invention comprises an indenoperylene derivative represented by the following formula (B-1):

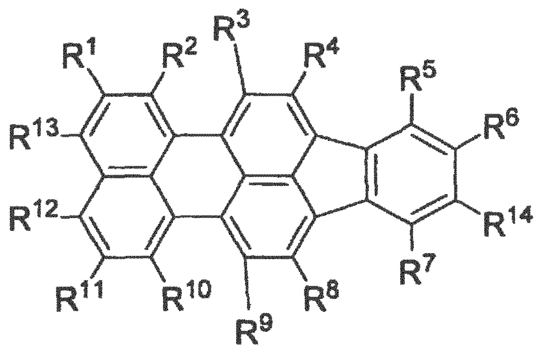

(B-1)

wherein $R^1$ to $R^{14}$ are independently an amino group presented by the formula (B-2), a hydrogen atom, a halogen atom, a substituted or unsubstituted alkyl group having 1 to 40 carbon atoms, a substituted or unsubstituted alkenyl group having 2 to 40 carbon atoms, a substituted or unsubstituted alkynyl group having 2 to 40 carbon atoms, a substituted or unsubstituted aryl group having 6 to 40 ring carbon atoms, a substituted or unsubstituted heteroaryl group having 5 to 40 ring atoms, a substituted or unsubstituted alkoxy group having 1 to 40 carbon atoms or a substituted or unsubstituted aryloxy group having 6 to 40 ring carbon atoms, and at least one of $R^1$ to $R^{14}$ is an amino group represented by the following formula (B-2);

adjacent groups of $R^1$ to $R^{14}$ may be bonded with each other to form a ring;

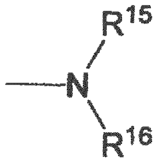

(B-2)

wherein $R^{15}$ and $R^{16}$ are independently a substituted or unsubstituted aryl group having 6 to 40 ring carbon atoms or a substituted or unsubstituted alkyl group having 1 to 40 carbon atoms.

As for the indenoperylene derivative represented by the formula (B-1) which is contained in the organic thin film solar cell material according to the second aspect of the invention (hereinafter often simply referred to as the second indenoperylene derivative), in respect of productivity, it is preferred that it have a structure in which a fluoranthene skeleton is provided only on one side of a perylene skeleton, whereby an increase in molecular weight is suppressed.

The structure of the indenoperylene derivative of the invention is also a structure in which the $\pi$-electron conjugated system is expanded to allow the absorption maximum wavelength to be shifted to a long wavelength side.

The second indenoperylene derivative which simultaneously has the above-mentioned structures can obtain a high conversion efficiency when used as the p material of the organic thin film solar cell. Further, when a cell is produced by the vapor evaporation method, a lowering in productivity caused by difficulty in sublimation can be eliminated.

In the formula (B-1), at least one of $R^1$ to $R^{14}$ is an amino group represented by the formula (B-2). Due to the presence of a diarylamino group a dialkylamino group or the like represented by the formula (B-2) having excessive electrons, in the second indenoperylene derivative, hole-transportation properties are enhanced, and conversion efficiency can be improved when used as a material of an organic thin film solar cell.

In respect of easiness in production, in the second indenoperylene derivative, it is preferred that at least one of $R^6$, $R^{12}$ , $R^{13}$ and $R^{14}$ be an amino group represented by the formula (B-2), and $R^1$ to $R^5$ and $R^7$ to $R^{11}$ are a substituent other than an amino group represented by the formula (B-2).

In respect of stable charge transfer and productivity, the number of the amino group represented by the formula (B-2) is preferably 1.

The second indenoperylene derivative is an indenoperylene derivative represented by the following formula (B-3) in which one of $R^8$ and $R^{14}$, which are the site into which an amino group is introduced easily, is an amino group represented by the formula (B-2):

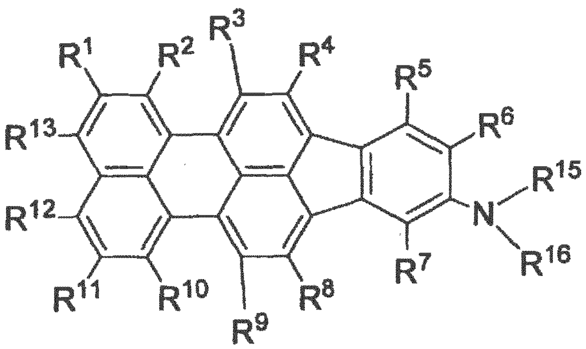

(B-3)

wherein $R^1$ to $R^{13}$, $R^{15}$ and $R^{16}$ are the same as those in the formulas (B-1) and (B-2).

Hereinbelow, an explanation will be made on each substituent of the second indenoperylene derivative.

As the halogen atom represented by $R^1$ to $R^{14}$, a fluorine atom, a chlorine atom, a bromine atom and an iodine atom can be given.

The substituted or unsubstituted alkyl group having 1 to 40 carbon atoms represented by $R^1$ to $R^{14}$, $R^{15}$ and $R^{16}$ may be linear, branched or cyclic. Specific examples thereof include a methyl group, an ethyl group, a 1-propyl group, a 2-propyl group, a 1-butyl group, a 2-butyl group, a sec-butyl group, a tert-butyl group, various pentyl groups, various hexyl groups, various octyl groups, various decyl groups, various dodecyl groups, a 2-ethylhexyl group, a 3,7-dimethyloctyl group, a cyclopropyl group, a cyclopentyl group, a cyclohexyl group, a 1-adamantyl group, a 2-adamantyl group, a norbornyl group, a trifluoromethyl group, a trichloromethyl group, a benzyl group, an $\alpha$, $\alpha$-dimethylbenzyl group, a 2-phenylethyl group and a 1-phenylethyl group or the like. Of these, in respect of easiness in obtaining a raw material, an alkyl group having 1 to 20 carbon atoms is preferable, with a methyl group, an ethyl group, a 1-propyl group, a 2-propyl group, a tert-butyl group and a cyclohexyl group being further preferable.

As the substituent for the above-mentioned alkyl group, a halogen atom such as a fluorine atom, a chlorine atom, a

bromine atom and an iodine atom, and an aryl group such as a phenyl group can be given. The aryl group may be further substituted by an alkyl group having 1 to 5 carbon atoms such as a methyl group, an ethyl group and a propyl group.

The substituted or unsubstituted alkenyl group having 2 to 40 carbon atoms represented by $R^1$ to $R^{14}$ may be linear, branched or cyclic. Specific examples thereof include a vinyl group, a propenyl group, a butenyl group, an oleyl group, an eicosapentaenyl group, a docosahexaenyl group, a styryl group, a 2,2-diphenylvinyl group, a 1,2,2-triphenylvinyl group, and a 2-phenyl-2-propenyl group or the like. Of these, in respect of easiness in obtaining a raw material or for other reasons, an alkenyl group having 2 to 20 carbon atoms is preferable, with a vinyl group, a styryl group and a 2,2-diphenylvinyl group being further preferable.

As the substituent for the above-mentioned alkenyl group, a halogen atom such as a fluorine atom, a chlorine atom, a bromine atom and an iodine atom, an aryl group such as a phenyl group, and an alkyl group having 1 to 5 carbon atoms such as a methyl group, an ethyl group, and a propyl group can be given.

The substituted or unsubstituted alkynyl group having 2 to 40 carbon atoms represented by $R^1$ to $R^{14}$ may be linear, branched or cyclic. Specific examples thereof include an etenyl group, a propynyl group and a 2-phenyletenyl group. In respect of easiness in obtaining a raw material or for other reasons, an alkynyl group having 2 to 20 carbon atoms is preferable.

As the substituent for the above-mentioned alkenyl group, a halogen atom such as a fluorine atom, a chlorine atom, a bromine atom and an iodine atom, an aryl group such as a phenyl group, and an alkyl group having 1 to 5 carbon atoms such as a methyl group, an ethyl group, and a propyl group can be given.

Specific examples of the substituted or unsubstituted aryl group having 6 to 40 ring carbon atoms represented by $R^1$ to $R^{14}$, $R^{15}$ and $R^{16}$ include a phenyl group, a 2-tolyl group, a 4-tolyl group, a 4-trifluoromethylphenyl group, a 4-methoxyphenyl group, a 4-cyanophenyl group, a 2-biphenylyl group, a 3-biphenylyl group, a 4-biphenylyl group, a terphenylyl group, a 3,5-diphenylphenyl group, a 3,4-diphenylphenyl group, a pentaphenyphenyl group, a 4-(2,2-diphenylvinyl)phenyl group, a 4-(1,2,2-triphenylvinyl)phenyl group, a fluorenyl group, a 1-naphthyl group, a 2-naphthyl group, a 9-anthryl group, a 2-anthryl group, a 2-(1,4-diphenyl) anthryl group, a 2-(9,10-diphenyl) anthryl group, a 9-phenanthryl group, a 1-pyrenyl group, a chrycenyl group, a naphthacenyl group, and a coronenyl group. Of these, an aryl group having 6 to 30 ring carbon atoms is preferable in respect of easiness in obtaining a raw material or for other reasons, with a phenyl group, a 4-biphenyl group, a 1-naphthyl group, a 2-naphthyl group, a 2-anthryl group, a 9-phenanthryl group or the like being further preferable.

As the substituent for the aryl group, for example, a halogen atom such as a fluorine atom, a chlorine atom, a bromine atom and an iodine atom, an alkyl group having 1 to 5 carbon atoms such as a methyl group, an ethyl group and a propyl group, an alkoxy group having 1 to 5 carbon atoms such as a methoxy group, an ethoxy group and a propoxy group, a cyano group, an aryl group such as a phenyl group, an aryl group such as a phenyl group can be given.

In $R^{15}$ and $R^{16}$ in the formula (B-2), stable charge transfer can be obtained due to the stable presence of radical cations (holes) in an aryl group rather than in an alkyl group. Therefore, it is preferred that both $R^{15}$ and $R^{18}$ be both an aryl group. As examples of the aryl group, a phenyl group, a 1-naphthyl group, a 2-naphthyl group, a biphenyl group, a 9-phenanthryl group, a 1-pyrenyl group or the like can be given.

As the heteroaryl group having 5 to 40 ring atoms represented by $R^1$ to $R^{14}$, a monovalent residue corresponding to furan, thiophene, pyrrole, imidazole, benzimidazole, pyrazole, benzpyrazole, triazole, oxadiazole, pyridine, pyrazine, triazine, quinoline, benzofuran, dibenzofuran, benzothiophene, dibenzothiophene and carbazole can be given. Of these, in respect of easiness in obtaining a raw material or for other reasons, furan, thiophen, pyridine, carbazole or the like are preferable.

As the substituent for the above-mentioned heteroaryl group, a halogen atom such as a fluorine atom, a chlorine atom, a bromine atom and an iodine atom and an alkyl group having 1 to 5 carbon atoms such as a methyl group, an ethyl group and a propyl group can be given.

If the above-mentioned heteroaryl group is a monovalent residue corresponding to the nitrogen-containing azole-based heterocyclic ring, it may be bonded to the indenoperylene skeleton represented by the formula (B-1) through carbon or nitrogen.

The substituted or unsubstituted alkoxy group having 1 to 40 carbon atoms represented by $R^1$ to $R^{14}$ may be linear, branched or cyclic. Examples thereof include a methoxy group, an ethoxy group, a 1-propyloxy group, a 2-propyloxy group, a 1-butyloxy group, a 2-butyloxy group, a sec-butyloxy group, various tert-butyloxy group, a pentyloxy group, various hexyloxy groups, various octyloxy groups, various decyloxy groups, various dodecyloxy groups, a 2-ethylhexyloxy group, a 3,7-dimethyloctyloxy group, a cyclopropyloxy group, a cyclopentyloxy group, a cyclohexyloxy group, a 1-adamantyloxy group, a 2-adamantyloxy group, a norbomyloxy group, a trifluoromethoxy group, a benzyloxy group, an $\alpha,\alpha$-dimethylbenzyloxy group, a 2-phenylethoxy group and a 1-phenylethoxy group. Of these, in respect of easiness in obtaining raw materials or for other reasons, an alkoxy group having 1 to 20 carbon atoms is preferable, with a methoxy group, an ethoxy group, a tert-butyloxy group and the like being further preferable.

As the substituent for the above-mentioned alkoxy group, for example, a halogen atom such as a fluorine atom, a chlorine atom, a bromine atom and an iodine atom; and an aryl group such as a phenyl group can be given. The aryl group may

further be substituted by an alkyl group having 1 to 5 carbon atoms such as a methyl group, an ethyl group and a propyl group.

As the aryloxy group having 6 to 40 carbon atoms represented by $R^1$ to $R^{14}$, for example, a substituent in which an oxygen atom is further bonded to the aryl group can be given. In respect of easiness in obtaining a raw material or for other reasons, a phenoxy group, a naphthoxy group, a phenanthryloxy group or the like are preferable.

The adjacent groups of $R^1$ to $R^{14}$ may be bonded with each other to form a ring. However, there may be a case where $R^6$ and $R^{14}$ are not bonded with each other to form a ring.

Examples of the ring include an aliphatic ring such as a pentane ring, an aryl ring such as a benzene ring, a heterocyclic ring such as a thiophene ring, and a fused ring formed of these groups (a naphthalene ring, an anthracene ring, a benzothiophene ring or the like). The ring may have a group which is exemplified as above as $R^1$ to $R^{14}$ groups as a substituent. As examples in which a ring is formed, the compounds represented by the following formula can be given.

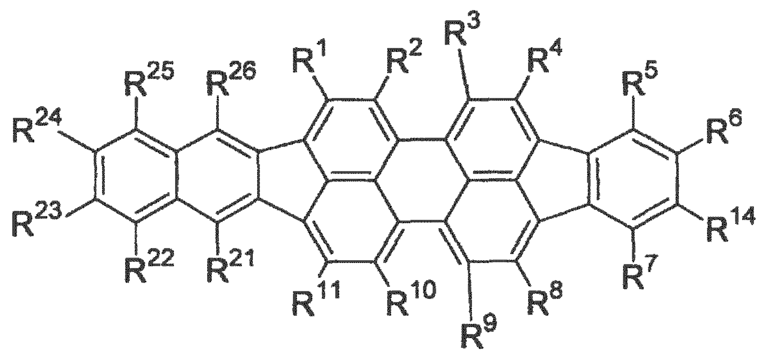

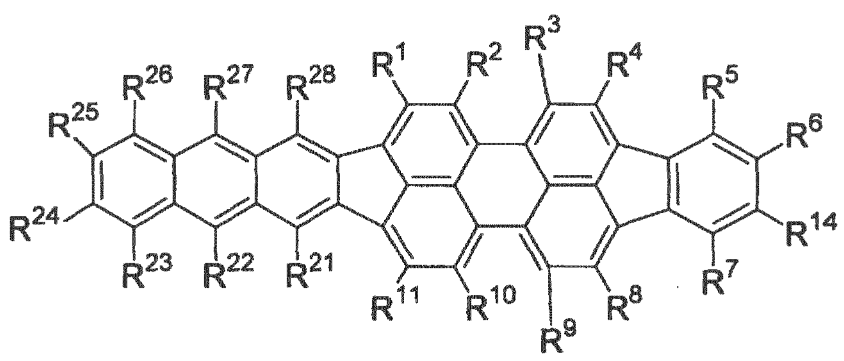

(in the formulas, $R^1$ to $R^{28}$ are independently the same groups as those for $R^1$ to $R^{14}$ mentioned above)

Specific examples of the second indenoperylene derivative of the invention are given below.

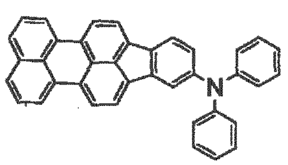
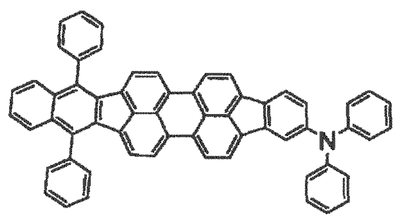
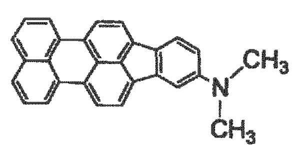

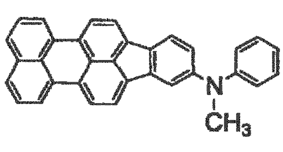
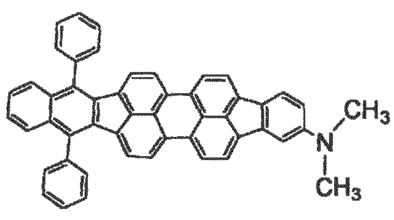
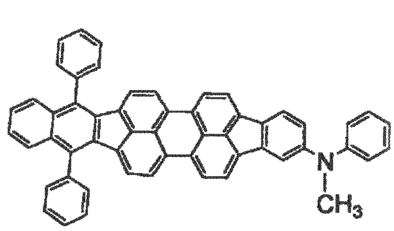

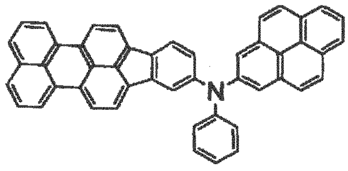
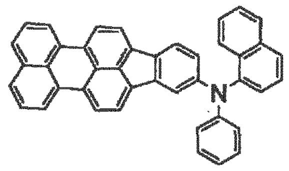
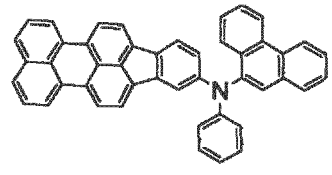

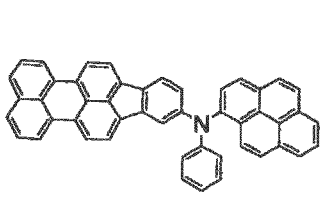
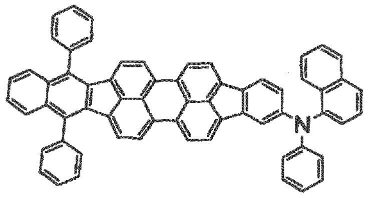
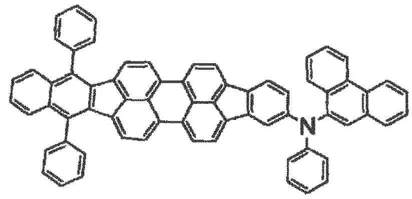

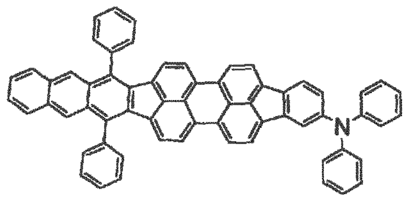
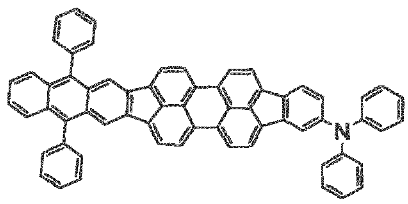

As the method for synthesizing the second indenoperylene derivative, in respect of easiness in obtaining a raw material, moderate reaction conditions and a high yield or the like, a synthesis route in which a perylene derivative is subjected to a ring-closing reaction is preferable.
An example of the synthesis route of the second indenoperylene derivative in which a perylene derivative is subjected to a ring-closing reaction is shown below.

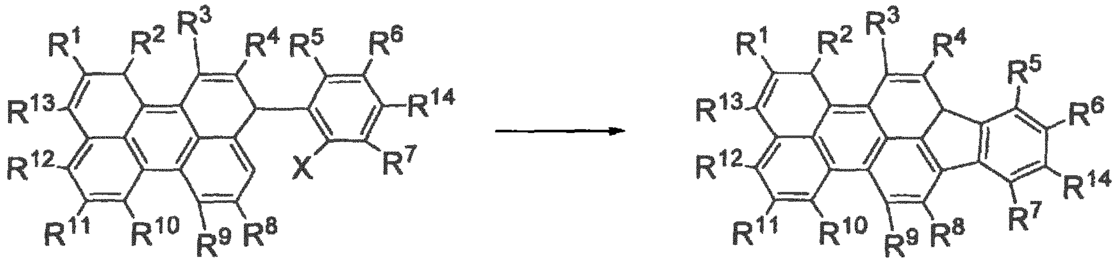

In the above-mentioned synthesis route, X as the starting material is a halogen atom such as an iodine atom, a bromine atom and a chlorine atom; ora pseudohalogen atom such as a trifluromethanesulfonyloxy(trifluoroxy) group, a nonafluorobutenesulfonyloxy(nonafuryloxy) group, a toluenesulfonyloxy(tosyloxy) group and a methansulfonyloxy(mesyloxy) group. Of these, in respect of easiness in obtaining a raw material or for other reasons, it is preferred that X be a halogen atom such as a chlorine atom and a bromine atom.
Further, in respect of easiness in obtaining a raw material, capability of synthesis with a small number of synthesis steps, and a high yield at moderate reaction conditions or for other reasons, regarding the substitution position of the amino group represented by the formula (B-2) as the starting material, it is preferred that the substitution position be $R^6$ or $R^{14}$.
As the catalyst used in the ring-closing reaction, a combination of various metals and various ligands can be used. As for the type of a metal, nickel or palladium is preferable since a high yield can be obtained. For example, nickel chloride, dichlorobis(tripheny)phosphine)nickel, tetrakis(triphenylphosphine)nickel, bis(cyclooctadiene)nickel, nickel tetracarbonyl, bis(acetylacetonate)nickel, nickelocene, tetrakis(triphenylphosphine)palladium, dichlorobis(triphenylphosphine)palladium, tris(dibenzylideneacetone)dipalladium, palladium acetate, palladium chloride or the like can be given.
As for the ligand, phosphines such as triphenylphosphine, tri-o-tolylphosphosphine, 2,2'-bis(diphenylphosphino)-1,1'-binaphthyl (BINAP), 4,5-bis(diphenylphosphino)-9,9-dimethylxanthene (XantPhos), 1,2-bis(diphenylphosphino)benzene, 1,1'-bis(diphenylphosphino)ferrocene (DPPF), tri-t-butylphosphine, 2-(dicyclohexylphosphino)biphenyl (JohnPhos), 2-(dicyclohexylphosphino)-2'-(N,N-dimethylamino)biphenyl (DavePhos), 2-(2-dicyclohexylphosphino)-2',6'-dimethoxybiphenyl (S-Phos) and 2-(2-dicyclohexylphoshino)-2',4',6'-triisopropylbiphenyl (X-Phos) are preferable.
It is preferable to add a base at the time of a ring-closing reaction. As the base, a carbonate such as potassium

carbonate and cesium carbonate; an alkoxide such as sodium t-butoxide and an organic base such as diazabicycloundecene, diazabicyclononene, triethylamine, diisopropylethylamine (Huening's base), pyridine and 4-dimethylaminopyridine (DMAP) can be given. Due to the possibility of obtaining a high yield, an organic base such as diazabicycloundecene is preferable.

In order to increase the reactivity of the base, it is further preferable to add a phase transfer catalyst. As the phase transfer catalyst, tetrabutylammonium hydrogensulfate, benzyltriethylammonium chloride or the like can be given.

The first and second indenoperylene derivatives of the invention can be preferably used as the material for an organic thin film solar cell. In particular, it can be used preferably as a material for the p layer of an organic thin film solar cell. The reason therefor is considered to be as follows. Due to a long wavelength absorption based on a perylene skeleton, affinity with a solar light spectrum of a solar cell is increased, whereby excitons tend to be generated easily. Holes generated by charge separation of the excitons tend to be transported easily to an electrode due to the amine structure constituting an indenoperylene derivative.

The material for an organic thin film solar cell of the invention comprises at least one of the first indenoperylene derivative and the second indenoperylene derivative of the invention. The material for an organic thin film solar cell of the invention may be composed only of the first indenoperylene derivative and/or the second indenoperylene derivative, or may be a mixture which further contains other known components.

A solar cell using the organic thin film solar cell material of the invention can exhibit highly efficient conversion properties.

No particular restrictions are imposed on the cell structure of the organic thin film solar cell of the invention as long as it is a structure in which the organic thin film solar cell material of the invention is contained between a pair of electrodes. Specifically, structures in which the following constitutional elements (1) to (3) are provided on a stable insulating substrate can be given.

(1) Lower electrode/p layer/n layer/Upper electrode
(2) Lower electrode/p layer/i layer (or a mixture layer of a p material and an n material)/n layer/Upper electrode
(3) Lower electrode/a mixture layer of a p material and an n material/ Upper electrode

In the above-mentioned cell structure, the p layer and the n layer may be replaced.

Moreover, a buffer layer may be provided between an electrode and an organic layer, if necessary.

As the cell structure having a buffer layer, a structure having the following constitutional elements (4) to (6) can be given.

(4) Lower electrode/Buffer layer/ p layer/n layer/Upper electrode
(5) Lower electrode/p layer/n layer/Buffer layer/Upper electrode
(6) Lower electrode/Buffer layer/p layer/n layer/Buffer layer/Upper electrode

The material for an organic thin film solar cell of the invention can be preferably used in the material for the p layer and the i layer. Further, if the first and the second indenoperylene derivatives of the invention have an electron-attracting group such as a fluorine atom, it can be used as the material for the n layer and the buffer layer.

In the organic thin film solar cell of the invention, it suffices that the compound of the invention be contained in any of the elements constituting the cell. Further, the components containing the compound of the invention may contain other known components in combination. As for the constitutional elements which do not contain the compound of the invention, it is possible to use a known material which is used in an organic thin film solar cell. The organic thin film solar cell of the invention may be one which is obtained by stacking a plurality of units each composed of the p layer, the i layer and the n layer.

Each constitutional element of the organic thin film solar cell will be briefly explained below.

[Lower electrode and upper electrode]

No particular restrictions are imposed on the material for the lower electrode and the upper electrode, and a known conductive material can be used.

For example, as the material for the electrode connecting with the p layer, a metal such as tin-doped indium oxide (ITO), gold (Au), osmium (Os) and palladium (Pd) or the like can be used.

As the material for the electrode connecting with the n layer, metals such as silver (Ag), aluminum (Al), indium (In), calcium (Ca), platinum (Pt) and lithium (Li); a binary metal system such as Mg:Ag, Mg:In or Al:Li, and the above-mentioned materials for the electrode connecting with the p layer can be used.

As for the pair of electrodes, one of the electrodes contains a metal having a large work function and the other contains a metal having a small work function.

To obtain highly efficient photoelectric conversion properties, it is desired that at least one electrode of the organic thin film solar cell preferably have sufficient transparency in the solar light spectrum.

The transparent electrode can be formed of a known conductive material by deposition, sputtering or the like such that the predetermined light transmittance is secured. It is preferred that the light transmittance of the electrode on the light-receiving surface be 10% or more.

[Organic compound layer]

The organic compound layer is the p layer, a mixture layer of a p material and an n material or the n layer. When the compound of the invention is used in the organic compound layer, specfically, a configuration having the lower electrode and a single layer of the compound of the invention and the upper layer, or a configuration having the lower electrode, a mixture layer of the material of the invention and the n layer material or the p layer material, mentioned later and the upper electrode, or the like can be given.

[p layer, n layer and i layer]

As the material for the p layer, a material having a function as a hole acceptor is preferable.
For example, amine compounds represented by N,N'-bis(3-tolyl)-N,N'-diphenylbenzidine (mTPD), N,N'-dinaphthyl-N, N'-diphenylbenzidine (NPD) and 4,4',4"-tris(phenyl-3-tolylamino)triphenylamine (MTDATA); phthalocyanines such as phthalocyanine (Pc), copper phthalocyanine (CuPc), zinc phthalocyanine (ZnPc) and titanylphthalocyanine (TiOPc); and porphyrins represented by octaethylporphyrin (OEP), platinum octaethylporphyrin (PtOEP) and zinc tetraphenylporphyrin (ZnTPP); main chain-type conjugated polymers such as polyhexylthiophene (P3HT) and methoxyethylhexyloxyphenylenevinylene (MEHPPV), and side chain-type polymers represented by polyvinyl carbazole can be given.
As the material for the n layer, a material having a function as an electron acceptor is preferable. More preferred are compounds having high electron mobility. Preferred are materials having a small electron affinity. Use of such a material having a small electron affinity can lead to a sufficient open-circuit voltage.
As for the material for the n layer, as organic compounds, fullerene derivatives such as $C_{60}$, $C_{70}$, carbon nanotube, perylene derivatives, polycyclic quinones and quinacridone can be given, and as polymers, CN-poly(phenylene-vinylene), MEH-CN-PPV, polymers having a -CN group or a $CF_3$ group, those polymers substituted by a $-CF_3$ group and poly(fluorene)derivatives or the like can be given.
As for the material for the n layer, as inorganic compounds, an inorganic semiconductor compound having n-type characteristics can be given. Specific examples include doped semiconductors and compound semiconductors such as n-Si, GaAs, CdS, PbS, CdSe, InP, $Nb_2O_5$, $WO_3$ and $Fe_2O_3$; and titanium oxides such as titanium dioxide ($TiO_2$), titanium monoxide (TiO) and dititanium trioxide ($Ti_2O_3$); and conductive oxides such as zinc oxide (ZnO) and tin oxide ($SnO_2$). The above-mentioned inorganic semiconductor compounds having an n-type characteristic may be used alone or in combination of two or more. Of these, titanium oxide is preferabe used, with titanium dioxide being particularly preferable.
The material for an i layer is a mixture of the material for the p layer and the material for the n layer. The above-mentioned materials can be used.
When of the invention is used as the material for the i layer, the above-mentioned material for the p layer or the above-mentioned material for the n layer may be mixed. It is possible to use the material for an organic thin film solar cell of the invention singly as the i layer.

[Buffer layer]

In general, an organic thin film solar cell has a small total film thickness, and hence, the upper electrode and the lower electrode often short-circuit so that a yield in fabrication of the cells may decrease. Such short-circuit can be avoided by stacking a buffer layer.
As materials for forming the buffer layer, preferred are compounds having a sufficiently high carrier mobility such that the short-circuit current does not decrease even when the film thickness of the buffer layer increases. Examples of the material for the buffer layer include, as a low molecular compound, aromatic cyclic acid anhydrides represented by NTCDA as shown below, and as a polymer compound, known conductive polymers represented by poly(3,4-ethylenedioxy)thiophene:polystyrene sulfonate (PEDOT:PSS) and polyaniline:camphor sulfonic acid (PANI:CSA).

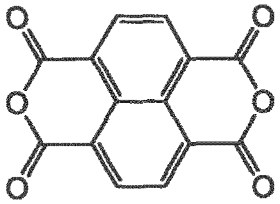

NTCDA

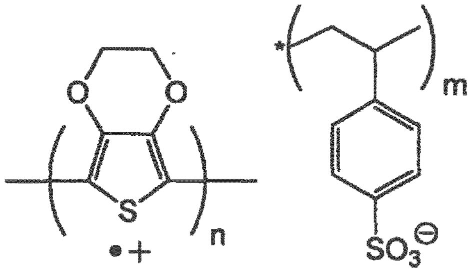

PEDOT:PSS

The buffer layer may have a role of preventing excitons from deactivation due to diffusion to the electrode. It is effective for enhancing the efficiency that the buffer layer is inserted as an exciton blocking layer. The exciton blocking layer may be inserted into each of the anode side and the cathode side, and may also be inserted into both the sides at the same time.

Preferred materials for the buffer layer as an exciton blocking layer include known materials for the hole barrier layer and for the electron barrier layer in organic EL devices.

Preferred materials for the hole barrier layer are compounds having a sufficiently large ionization potential. Preferred materials for the electron barrier layer are compounds having a sufficiently small electron affinity. Specifically, bathocuproin (BCP), bathophenanthroline (BPhen) and the like, can be given as the material for the hole barrier layer on the cathode side.

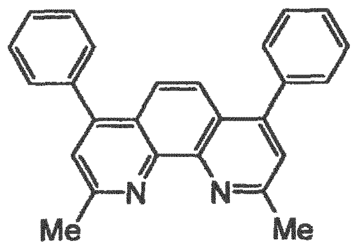

BCP

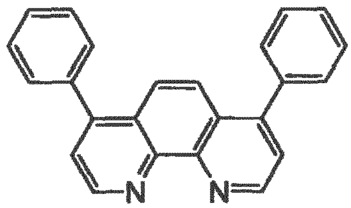

BPhen

In addition to the above-mentioned compounds, the inorganic semiconductor compounds exemplified as the materials for the n layer as mentioned above may be used as the materials for the buffer layer. Inorganic semiconductor compounds such as CdTe, p-Si, SiC, GaAs and $WO_3$ can be used.

[Substrate]

As the material for the substrate, materials having mechanical strength, thermal strength and transparency are preferable. Examples of the substrate include glass substrates and transparent resin films. The transparent resin films include films made of polyethylene, ethylene-vinyl acetate copolymer, ethylene-vinylalcohol copolymer, polypropylene, polystyrene, poly(methyl methacrylate), polyvinylchloride, polyvinylalcohol, polyvinylbutyral, nylon, polyether ether ketone, polysulfone, polyether sulfone, tetrafluoroethylene-perfluoroalkylvinyl ether copolymer, polyvinylfluoride, tetrafluoroethylene-ethylene copolymer, tetrafluoroethylene-hexafluoropropylene copolymer, polychlorotrifluoroethylene, polyvinylidene fluoride, polyester, polycarbonate, polyurethane, polyimide, polyetherimide, polyimide and polypropylene.

Each layer of the organic thin film solar cell of the invention can be formed by dry film-forming methods such as vacuum vapor deposition, sputtering, plasma coating, and ion plating, and wet film-forming methods such as spin coating, dip coating, casting, roll coating, flow coating and inkjet.

The film thickness of each layer is not particularly limited, but the film can be made into an appropriate film thickness. It is generally known that the exciton diffusion length of an organic thin film is short. If the film thickness is too thick, excitons may be deactivated before they reach the hetero interface, so that photoelectric conversion efficiency becomes low. On the other hand, if the film thickness is too thin, generation of pinholes or other problems may occur. As a result, the sufficient diode properties cannot be obtained, resulting in lowering of the conversion efficiency. The appropriate film thickness of each layer is usually in a range of 1 nm to 10 μm, and further preferably in a range of 5 nm to 0.2 μm.

In the case of using the dry film-forming method, known resistance heating methods are preferable. In the case of forming a mixture layer, a film-forming method in which co-deposition is conducted using plural evaporation sources is preferable, for example. Further preferred is to control the substrate temperature during film-formation.

In the case of employing the wet film-forming method, a material for forming each layer is dissolved or dispersed in an appropriate solvent to prepare an organic solution, and a thin film is formed from the solution. The solvent can be arbitrarily selected. The usable solvent includes halogenated hydrocarbon-based solvents such as dichloromethane, dichloroethane, chloroform, carbon tetrachloride, tetrachloroethane, trichloroethane, chlorobenzene, dichlorobenzene and chlorotoluene; ether solvents such as dibutyl ether, tetrahydrofuran, dioxane and anisole; alcohol solvents such as methanol, ethanol, propanol, butanol, pentanol, hexanol, cyclohexanol, methyl cellosolve, ethyl cellosolve and ethylene glycol; hydrocarbon solvents such as benzene, toluene, xylene, ethylbenzene, hexane, octane, decane and tetralin; and ester solvents such as ethyl acetate, butyl acetate and amyl acetate. Of these, the hydrocarbon-based solvents or the ether solvents are preferable. These solvents may be used singly or in a mixture of two or more. Usable solvents are not limited thereto.

In the invention, appropriate resins or additives may be used in any of the organic thin film layers in the organic thin film solar cell in order to improve film-forming properties or for the prevention of generating pinholes of the film, or the like. Usable resins include insulating resins such as polystyrene, polycarbonate, polyarylate, polyester, polyamide, polyurethane, polysulfone, poly(methyl methacrylate), poly(methyl acrylate) and cellulose, and copolymers thereof; photo-conductive resins such as poly-N-vinylcarbazole and polysilane; and conductive resins such as polythiophene and polypyrrole.
The additives include an antioxidant, an ultraviolet absorbent and a plasticizer.

## EXAMPLES

Example 1 (synthesis of compound A)

Raw material A for compound A was synthesized according to the following procedure.

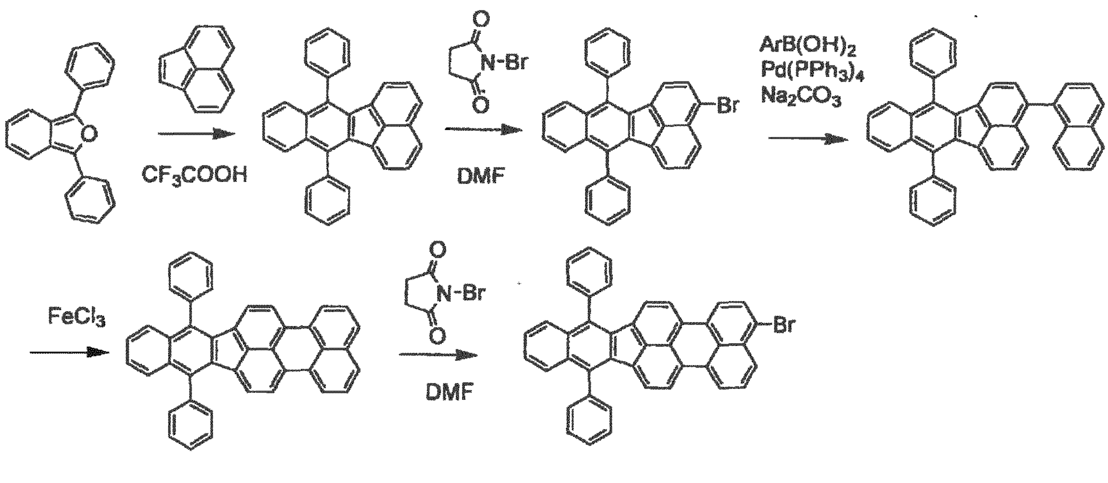


(Raw material A)

Compound A was synthesized as follows.

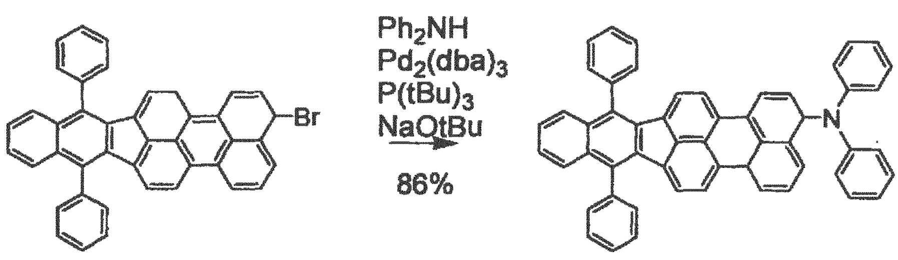


(Raw material A) (Compound A)

In the atmosphere of nitrogen, raw material A (1.2g, 8.9 mmol), diphenylamine (0.4g, 2.4 mmol, 1.2 eq.), tris(dibenzylideneacetone)dipalladium (0) (0.03g, 0.03 mmol, 3% Pd) and sodium t-butoxdie (0.3g, 2.8 mmol, 1.4 eq.) were suspended in toluene anhydride (50 ml). To the resulting suspension, a tri-t-butylphosphine/totuene solution (2.8 M, 0.017 ml, 0.048 mmol, 0.8 eq. relative to Pd) was added, followed by reflux for 7 hours. The reaction mixture was filtered out through a silica gel pad, and washed with toluene (100 ml). A red oily solid obtained by distilling off the solvent from the filtrate was purified by column chromatography (silica gel/hexane + 17% dichloromethane, subsequently hexane + 33% dichloromethane), whereby a red solid (1.2g, 86%) was obtained.

For the resulting red solid, various evaluations were conducted. The results are shown below. $^1$H-NMR (400MHz, $CDCl_3$, tetramethylsilane(TMS)) 6.68(2H,d,J=8Hz), 6.93 (2H,t,J=9Hz), 7.06-7.08 (4H,m), 7.19-7.42 (6H,m), 7.59-7.72 (12H,m), 7.86 (1H,d,J=9z), 7.95 (1H,d,J=8Hz), 7.99 (1H,d,J=8Hz), 8.17 (2H,d,J=9Hz), field desorption mass spectrometry (FDMS):calculated value $C_{54}H_{35}N$=698, actually measured value m/z=698 ($M^+$,100), high-speed liquid chromatography (HPLC):99.1% (UV254 area%), absorption maximum wavelength: 546 nm (dichloromethane), fluorescent maximum wavelength: 585 nm (dichloromethane)

By purification by sublimation of the solid (1.2g) as obtained above at 340°C/1.9 $\times 10^{-3}$ Pa, a red solid (0.9g) was obtained. HPLC: 95.7% (UV254 area%)

Example 2

[Fabrication of an organic thin film solar cell]

A glass substrate of 25 mm by 75 mm by 0.7 mm thick with an ITO transparent electrode was subjected to ultrasonic cleaning with isopropyl alcohol for 5 minutes, and cleaned with ultraviolet rays and ozone for 30 minutes. The substrate with transparent electrode lines thus cleaned was mounted on a substrate holder in a vacuum deposition apparatus. Compound A (p type material) was formed into a 30 nm-thick film by the resistance heating deposition at a deposition rate of 1Å/s so as to cover the surface of the substrate on which the transparent electrode lines were formed as a lower electrode.

Subsequently, on this film, $C_{60}$ (n-layer material) was formed into a 60 nm-thick film by the resistance heating deposition at a deposition rate of 1 Å/s. Then, as the buffer layer, a 10 nm-film of bathocuproin (BCP) was formed at a deposition rate of 1 A/s. Finally, metal Al was deposited on the buffer layer as the opposing electrode having a film thickness of 80 nm, whereby an organic thin film solar cell having an area of 0.5 $cm^2$ was formed. I-V characteristics were determined for the organic solar cell thus fabricated under a condition of AM 1.5 (light intensity: 100 $mW/cm^2$). The open-circuit voltage (Voc), the short-circuit current density (Jsc), the fill factor (FF value) and the conversion efficiency ($\eta$) are shown in Table 1.

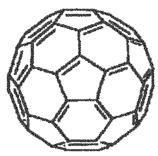

$C_{60}$

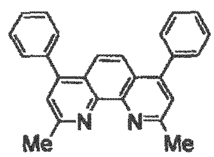

BCP

Comparative Example 1

An organic solar cell was fabricated in the same manner as in Example 2 except that mTPD was used instead of compound A. I-V characteristics were determined for the organic solar cell thus fabricated under a condition of AM 1.5 (light intensity: 100 $mW/cm^2$). The open-circuit voltage (Voc), the short-circuit current density (Jsc), the fill factor (FF value) and the conversion efficiency ($\eta$) are shown in Table 1.

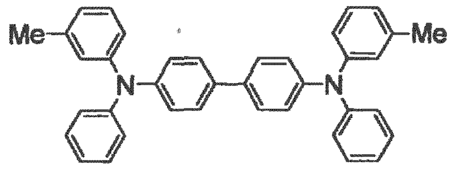

mTPD

Comparative Example 2

PFT was synthesized in the following procedure.

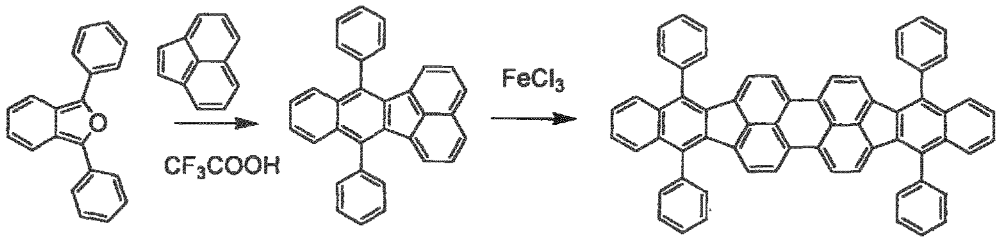


PFT

An organic solar cell was fabricated in the same manner as in Example 2 except that PFT was used instead of compound A. I-V characteristics were determined for the organic solar cell thus fabricated under a condition of AM 1.5 (light intensity: 100 mW/cm$^2$). The open-circuit voltage (Voc), the short-circuit current density (Jsc), the fill factor (FF value), the conversion efficiency (η) are shown in Table 1.

Comparative Example 3

RD was synthesized by the following procedure as described in JP-A-2009-132674.

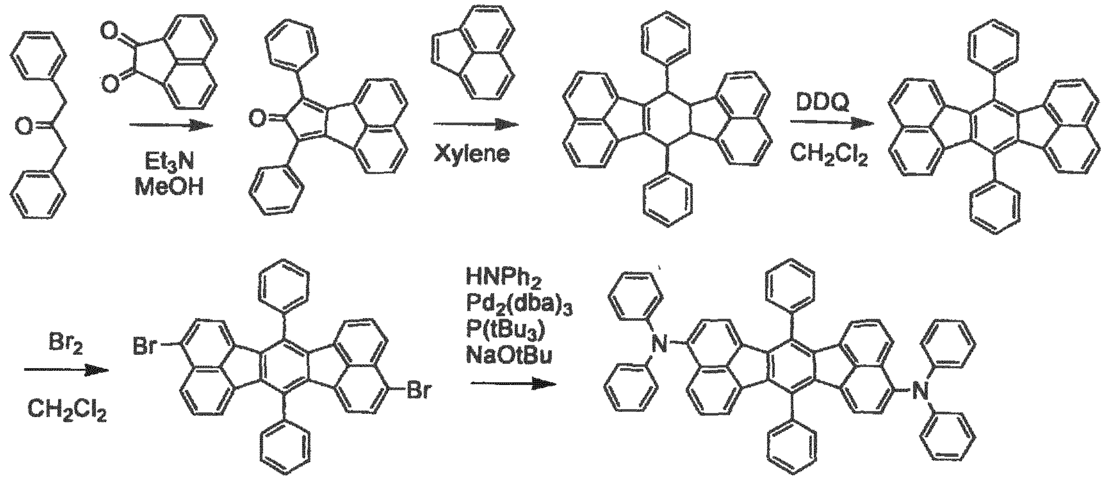


RD

An organic solar cell was fabricated in the same manner as in Example 2 except that RD was used instead of compound A. I-V characteristics were determined for the organic solar cell thus fabricated under a condition of AM 1.5 (light intensity: 100 mW/cm$^2$). The open-circuit voltage (Voc), the short-circuit current density (Jsc), the fill factor (FF value) and the conversion efficiency (η) are shown in Table 1.

TABLE 1

| | P Compound | N Compound | Voc [V] | Jsc [mA/cm$^2$] | FF | η [%] |
|---|---|---|---|---|---|---|
| Example 2 | Compound A | $C_{60}$ | 0.88 | 5.98 | 0.59 | 3.11 |
| Com. Ex. 1 | mTPD | $C_{60}$ | 0.71 | 0.71 | 0.34 | 0.17 |
| Com. Ex. 2 | PFT | $C_{60}$ | 0.91 | 4.78 | 0.40 | 1.72 |
| Com. Ex. 3 | RD | $C_{60}$ | 0.99 | 3.89 | 0.49 | 1.87 |

The photoelectric conversion efficiency (q) is generally represented by the following equation: The results are shown in Table 1.

$$\eta\ (\%) = \frac{Voc \times Jsc \times FF}{Pin} \times 100$$

wherein Voc is the open-circuit voltage, Jsc is the short-circuit current density, FF is the fill factor and Pin is the incident light energy.

From the above equation, it can be understood that a compound having a large Voc, a large Jsc and a large FF for the same Pin has an excellent conversion efficiency.

As is understood from Table 1, the compound of the invention has a higher conversion efficiency as compared with that of the conventional amine compound and polycyclic fused compound, and has excellent solar cell properties. The reason therefor is considered to be as follows. mTPD absorbs only light having a short wavelength and PFT does not have excellent hole-transporting properties. While RD can absorb light with a long wavelength and has hole-transporting properties, it has a narrower width of absorption of light with a longer wavelength as compared with the compound of the invention.

Example 3

[Synthesis of compound B]

Compound B was synthesized by the following synthesis route.

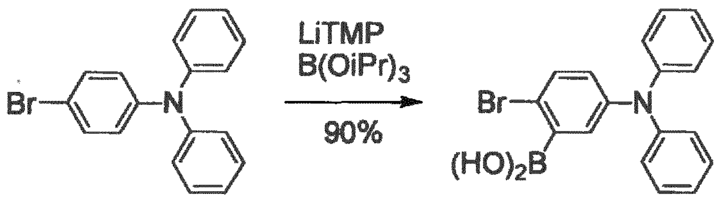


Raw material B

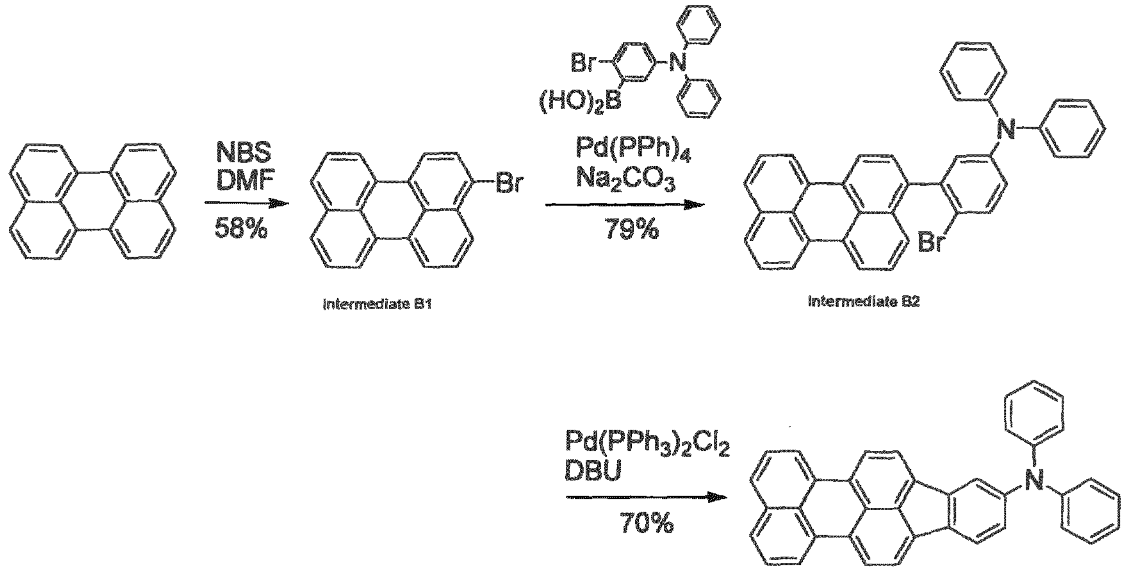


Intermediate B1

Intermediate B2

Compound B

[Synthesis of raw material B]

In the atmosphere of nitrogen, 2,2,6,6-tetramethylpiperidine (6.6g, 47 mmol, 1.5 eq.) was dissolved in anhydrous THF (60 ml). The resulting mixture was cooled to -43°C in a dry ice/methanol bath. To the resultant, an n-butyllithium/hexane solution (1.6 mol/l, 28ml, 47 mmol, 1 eq. to TMP) was added dropwise, and the resulting mixture was stirred at -23°C for 20 minutes. The reaction mixture was cooled to -74°C. Then, triisopropyl borate (15 ml, 65 mmol, 2 eq.) was added. After the lapse of 5 minutes, a 4-bromotriphenylamine/anhydrous THF solution (8g, 39 mmol/15ml) was added dropwise over 10 minutes. The reaction mixture was stirred at from -74°C to room temperature for 10 hours, and allowed to stand overnight. The reaction mixture was cooled in water bath. A 5% aqueous hydrochloric acid solution (100 ml) was gradually added to the mixture to allow it to be deactivated, and then diluted with ethyl acetate (150 ml). An organic phase was isolated, washed with saturated saline (30 ml) and dried with anhydrous magnesium sulfate. The solvent was distilled off, whereby yellow oil was obtained. The oil was purified by column chromatography (silica gel/hexane + 10% ethyl acetate, subsequently hexane + 17% ethyl acetate, hexane + 33% ethyl acetate), whereby a yellow solid (10.3g, 90%) was obtained.
The resulting solid was confirmed to be raw material B by NMR. The results are shown below. $^1$H-NMR (400MHz, $CDCl_3$, TMS) δ2.83 (2H,s), 6.92 (1H,dd,J=9Hz,3Hz), 7.04-7.08 (5H,m), 7.28-7.34 (5H,m), 7.41 (1H,d,J=8Hz)

[Synthesis of intermediate B1]

Perylene (5.0g, 20 mmol) was put in anhydrous DMF (200 ml), and the resulting mixture was heated to 100°C to dissolve perylene. Then, an anhydrous DMF solution (10 ml) of N-bromosuccinimide (3.5g, 20 mmol, 1.1 eq.) was added, followed by stirring at 100°C for 3 hours. The resultant was then allowed to stand for overnight. The reaction mixture was washed with methanol, whereby a yellow plate crystal (3.8g, 58%) was obtained.
The resulting crystal was confirmed to be intermediate B1 by NMR. The results are shown below.
$^1$H-NMR (400MHz, $CDCl_3$, TMS) δ7.45-7.51 (2H,m), 7.58 (1H,t,J=8Hz),7.70 (2H,d,J=8Hz), 7.76 (1H,d,J=8Hz), 7.99 (1H, d,J=8Hz), 8.08 (1H,d,J=8Hz), 8.14-8.24 (3H,m)

[Synthesis of intermediate B2]

In the atmosphere of nitrogen, raw material B (4.2g, 11 mmol), intermediate B1 (3.8g, 12 mmol) and tetrakis(triphenylphosphine)palladium (0) (0.25g, 0.22 mmol, 2%Pd) were dissolved in 1,2-dimethoxyethane (120 ml). Then, a 2M aqueous sodium carbonate solution (3.5g, 33 mmol, 3 eq./17 ml) was added, followed by reflux for 11 hours. The reaction mixture was diluted with water (100 ml), and filtered under reduced pressure to obtain a yellow solid. The solid was purified by column chromatography (silica gel/hexane + 5% dichloromethane, subsequently hexane + 10% dichloromethane), whereby a yellow plate crystal (5.0g, 79%) was obtained.
The resulting crystal was confirmed to be intermediate B2 by NMR. The results are shown below.
$^1$H-NMR (400MHz,$CDCl_3$,TMS) δ7.00-7.04 (3H,m), 7.11 (1H,d,J=3Hz), 7.15 (4H,d,J=7Hz), 7.24-7.28 (4H,m), 7.35 (1H,d,J=8Hz), 7.40-7.50 (2H,m), 7.55 (1H,d,J=8Hz), 7.69 (2H,d,J=8Hz), 8.20 (4H,d,J=8Hz)

[Synthesis of compound B]

In the atmosphere of nitrogen, intermediate B2 (5.0g, 8.7 mmol), dicyclobis(triphenylphosphine)palladium (II) (0.61g, 0.87 mmol, 10%Pd), 1,8-diazabicyclo[5.4.0]-7-undecene (2.0g, 13 mmol, 1.5 eq.) were dissolved in anhydrous DMF (150 ml), followed by stirring at 140°C for 11 hours. The reaction mixture was diluted with methanol (750 ml), filtered under reduced pressure to obtain a red blown solid. The solid was purified by column chromatography (silica gel/hexane + 33% dichloromethane, subsequently dichloromethane), whereby a red blown plate crystal (3.0g, 70%) was obtained. The resulting crystal was confirmed to be compound B by NMR. The results are shown below. $^1$H-NMR (400MHz, $CDCl_3$, TMS) $\delta$7.05 (2H,t,J=7Hz), 7.12 (1H,d,J=7Hz), 7.19 (4H,d,J=7Hz), 7.30 (4H,t,J=7Hz), 7.55-7.60 (2H,m), 7.70( 1H,s), 7.78-7.82 (3H,m), 7.87 (1H,d,J=7Hz), 7.92 (1H,d,J=7Hz), 8.27 (1 H,d,J=8Hz), 8.30 (1 H,d,J=8Hz), 8.37 (2H,d,J=8Hz)

For the resulting compound B, the purity thereof was measured by liquid chromatography to evaluate the absorption maximum wavelength and the fluorescence maximum wavelength. The results are shown below.

HPLC: 97.0% (UV 254 area%)
Absorption maximum wavelength: 487 nm (dichloromethane)
Fluorescence maximum wavelength: 654 nm (dichloromethane)

The resulting compound B (2.6 g) was purified by sublimation at 340°C/5.3 × $10^{-3}$ Pa, whereby an orange solid (2.5g) was obtained.

HPLC: 94.7% (UV 254 area%)

Example 4

[Synthesis of compound C]

Compound C was synthesized according to the following procedure.

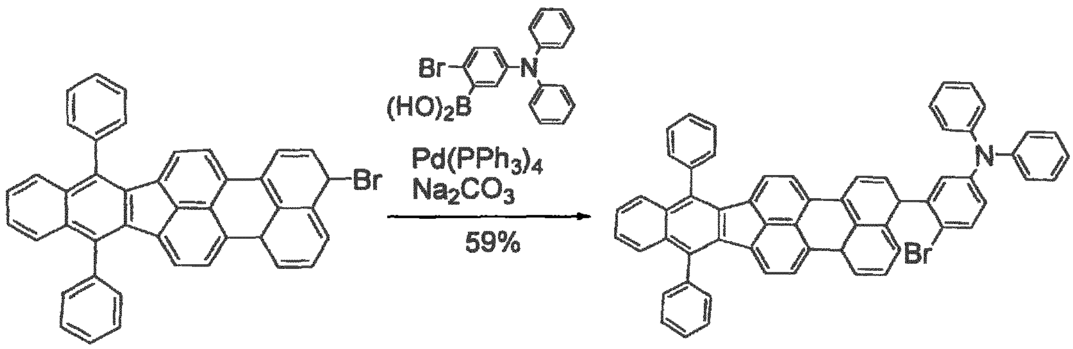


Raw material C

Intermediate C1

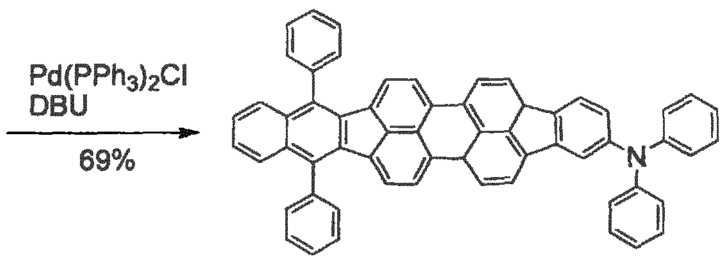


Intermediate C

[Synthesis of intermediate C1]

In the atmosphere of nitrogen, raw material C (2.0g, 3.3 mmol), raw material B (1.3g, 3.6 mmol, 1.1 eq.) and tetrakis(triphenylphosphine)palladium (0) (0.12g, 0.19 mmol, 3%Pd) were dissolved in 1,2-dimethoxyethane (33 ml). Then, a 2M aqueous sodium carbonate solution (1.0g, 10 mmol, 3 eq./5 ml) was added, followed by reflux for 8 hours. The reaction mixture was diluted with toluene (100 ml), and an organic phase was separated, washed with saturated

saline (50 ml), dried with anhydrous magnesium sulfate. The solvent was distilled off to obtain brown oil. The oil was then purified by column chromatography (silica gel/hexane + 20% dichloromethane), whereby an orange solid (1.6g, 59%) was obtained.
The resulting crystal was confirmed to be intermediate C1 by NMR. The results are shown below.
$^1$H-NMR (400MHz,$CDCl_3$,TMS) δ7.67 (2H,d,J=8Hz), 7.01 (3H,d,J=8Hz), 7.07 (1H,d,J=2Hz), 7.12-7.18 (6H,m), 7.35-7.42 (5H,m), 7.54 (1 H,d,J=8Hz), 7.53-7.68 (14H,m), 8.00 (2H,d,J=8Hz), 8.19 (2H,d,J=8Hz)

[Synthesis of compound C]

In the atmosphere of nitrogen, intermediate C1 (1.6g, 1.9 mmol), tetrakis(triphenylphosphine)palladium (0) (0.23g, 0.25 mmol, 5%Pd), 1,8-diazabicyclo[5.4.0]-7-undecene (0.4g, 2.6 mmol, 1.4 eq.) were dissolved in anhydrous DMF (10 ml), followed by stirring at 140°C for 12 hours. The reaction mixture was diluted with toluene (100 ml), and washed with water (50 ml) and saturated saline (25 ml), dried with anhydrous sodium sulfate. The solvent was distilled off to obtain blown oil. The oil was then purified by column chromatography (silica gel/hexane + 15% dichloromethane), whereby a purple solid (1.0g, 69%) was obtained.
The purity of the resulting solid was evaluated by HPLC. The results are shown below.
HPLC: 86.0% (UV 254 area%)
The resulting solid (1.0g) was purified by sublimation at 400°C/3.2 × $10^{-3}$ Pa, whereby a black solid (0.6g) was obtained. The resulting solid was confirmed to be compound C by NMR. The results are shown below. Further, the purity and the absorption maximum were evaluated. The results are shown below.
HPLC: 88.0% (UV 254 area%)
Absorption maximum wavelength ($CH_2Cl_2$): 600 nm
$^1$H-NMR (400MHz, $CDCl_3$, TMS) δ6.60 (2H,d,J=4.2Hz), 7.01-7.06 (3H,m), 7.17 (4H,d,J=6Hz), 7.28 (4H,t,J=6Hz), 7.38 (2H,d,J=2Hz), 7.57-7.77 (16H,m), 7.98 (2H,d,J=6Hz), 8.08 (2H,d,J=6Hz)

Example 5

[Production of organic solar cell]

A glass substrate of 25 mm by 75 mm by 0.7 mm thick with an ITO transparent electrode was subjected to ultrasonic cleaning with isopropyl alcohol for 5 minutes, and cleaned with ultraviolet rays and ozone for 30 minutes. The glass substrate with transparent electrode lines thus cleaned was mounted on a substrate holder in a vacuum deposition apparatus. A p-layer compound was formed into a 30 nm-thick film by the resistance heating deposition of compound B at a deposition rate of 1 Å/s to form a p layer having a thickness of 30 nm so as to cover the surface of the substrate on which the transparent electrode lines were formed as a lower electrode. Subsequently, on this compound B film, the n-layer compound was formed into a 60 nm-thick film by the resistance heating deposition of C60 at a deposition rate of 1 Å/s. Then, as the buffer layer, a 10 nm-film of bathocuproin (BCP) was formed at a deposition rate of 1 Å/s. Finally, metal Al was deposited on the buffer layer as the opposing electrode having a film thickness of 80 nm, whereby an organic solar cell (area of 0.5 $cm^2$) was formed.

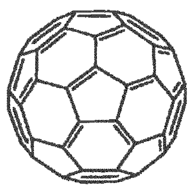
C60

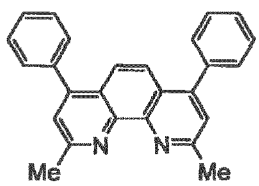
BCP

I-V characteristics were determined for the organic solar cell thus fabricated under a condition of AM 1.5 (light intensity: 100 $mW/cm^2$). The results, i.e. the open-circuit voltage (Voc), the short-circuit current density (Jsc), the fill factor (FF) and the conversion efficiency (η) are shown in Table 2.
The photoelectric conversion efficiency (η) was calculated by the following equation:

$$\eta\,(\%) = \frac{Voc \times Jsc \times FF}{Pin} \times 100$$

wherein Voc is the open-circuit voltage, Jsc is the short-circuit current density, FF is the fill factor and Pin is the incident light energy.
From the above equation, it can be understood that a compound having a large Voc, a large Jsc and a large FF for the same Pin has an excellent conversion efficiency.

Example 6

An organic solar cell was fabricated and evaluated in the same manner as in Example 5, except that the p layer was stacked using compound C instead of compound B. The results are shown in Table 2.

Comparative Example 4

An organic solar cell was fabricated and evaluated in the same manner as in Example 5, except that the p layer was stacked using the following compound A which was produced by the method described in Example 3 of WO2010/013520 instead of compound B. The results are shown in Table 2.

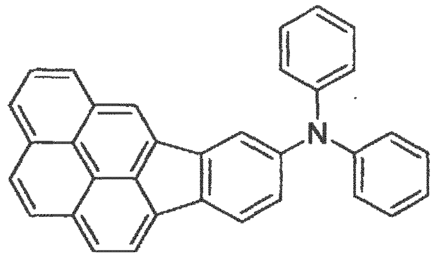

Comparative compound A

TABLE 2

| | P layer material | N layer material | Cell configuration | Voc [V] | Jsc [mA/cm$^2$] | FF [-] | η [%] |
|---|---|---|---|---|---|---|---|
| Example 5 | Compound B | $C_{60}$ | PN | 0.89 | 4.64 | 0.61 | 2.50 |
| Example 6 | Compound C | $C_{60}$ | PN | 0.91 | 5.40 | 0.59 | 2.91 |
| Com. Ex. 4 | Comparative Compound A | $C_{60}$ | PN | 0.93 | 4.01 | 0.65 | 2.40 |

From the results shown in Table 2, it can be confirmed that the solar cell produced by using the compounds B and C of Examples 3 and 4 of the invention had improved conversion efficiency as compared with the solar cell using conventional amine compounds (Comparative compound A), and had excellent solar cell properties.
The reason therefor is assumed to be as follows. While the comparative compound A had absorption of light with a long wavelength and hole-transporting properties, it had a narrower width of absorption of light with a longer wavelength as compared with the compound of the invention.

Industrial Applicability

The indenoperylene derivative of the invention and the material for an organic thin film solar cell of the invention can be used for an organic thin film solar cell and the organic thin film solar cell of the invention can be used as a power source of various devices or electrical appliances such as a watch, a cellular phone, a mobile PC or the like.
Although only some exemplary embodiments and/or examples of this invention have been described in detail above, those skilled in the art will readily appreciate that many modifications are possible in the exemplary embodiments and/or examples without materially departing from the novel teachings and advantages of this invention. Accordingly, all such modifications are intended to be included within the scope of this invention.
The documents described in the specification are incorporated herein by reference in its entirety.

## Claims

1. An indenoperylene derivative represented by a formula (A-1):

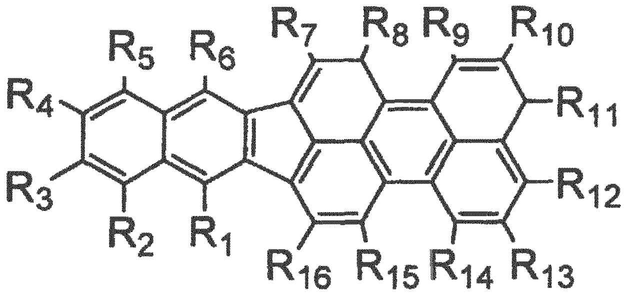

(A-1)

wherein in the formula (A-1), $R_1$ to $R_{16}$ are independently a hydrogen atom, a substituted or unsubstituted alkyl group having 1 to 40 carbon atoms, a substituted or unsubstituted alkenyl group having 2 to 40 carbon atoms, a substituted or unsubstituted alkynyl group having 2 to 40 carbon atoms, a substituted or unsubstituted aryl group having 6 to 40 ring carbon atoms, a substituted or unsubstituted heteroaryl group having 5 to 40 ring atoms, a substituted or unsubstituted alkoxy group having 1 to 40 carbon atoms, a substituted or unsubstituted aryloxy group having 6 to 40 ring carbon atoms or an amino group represented by the formula (A-2), and at least one of $R_1$ to $R_{16}$ is an amino group represented by the formula (A-2):

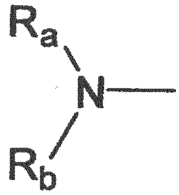

(A-2)

wherein $R_a$ and $R_b$ are independently a substituted or unsubstituted aryl group having 6 to 40 ring carbon atoms or a substituted or unsubstituted alkyl group having 1 to 40 carbon atoms.

2. The indenoperylene derivative according to claim 1, which is represented by a formula (A-3):

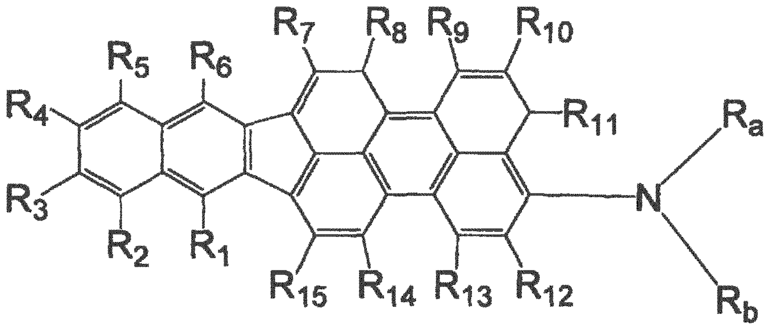

(A-3)

wherein in the formula (3), $R_1$ to $R_{15}$ and $R_a$ and $R_b$ are the same as those in the formulas (A-1) and (A-2).

3. The indenoperylene derivative according to claim 1 or 2, which is represented by a formula (A-4):

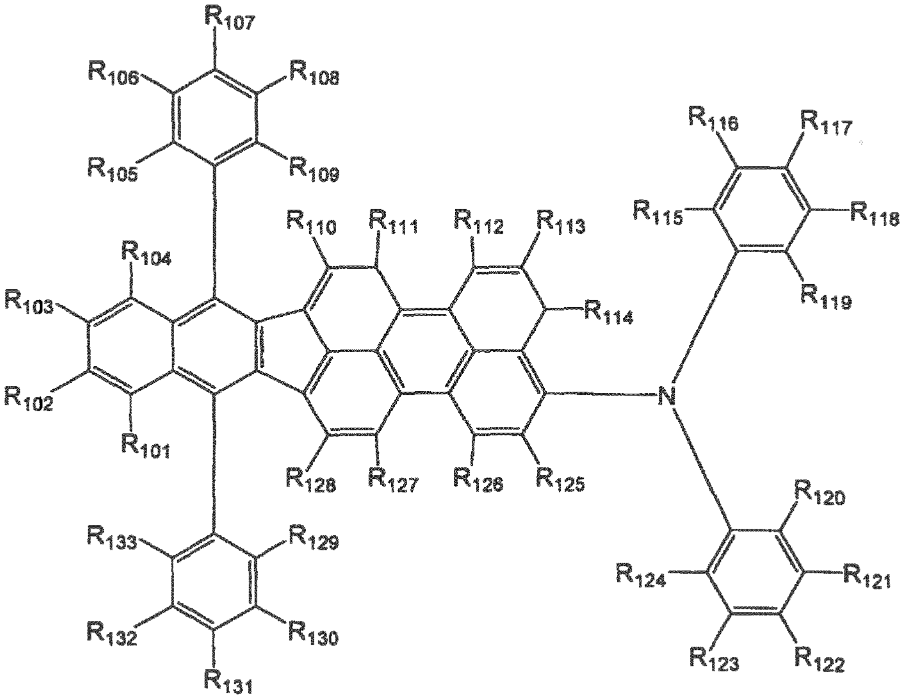

(A-4)

wherein $R_{101}$ to $R_{133}$ are independently a hydrogen atom, a substituted or unsubstituted alkyl group having 1 to 40 carbon atoms, a substituted or unsubstituted aryl group having 6 to 40 ring carbon atoms, a substituted or unsubstituted heteroaryl group having 5 to 40 ring atoms or a substituted or unsubstituted alkoxy group having 1 to 40 carbon atoms.

4. A material for an organic thin film solar cell comprising the indenoperylene derivative according to any of claims 1 to 3.

5. An organic thin film solar cell comprising the indenoperylene derivative according to any of claims 1 to 3.

6. An organic thin film solar cell comprising at least a p layer, wherein the p layer comprises the indenoperylene derivative according to any of claims 1 to 3.

7. An organic thin film solar cell comprising at least a p layer and an n layer, wherein the n layer comprise a fullerene derivative and the p layer comprises the indenoperylene derivative according to any of claims 1 to 3.

8. A material for an organic thin film solar cell comprising an indenoperylene derivative represented by the following formula (B-1):

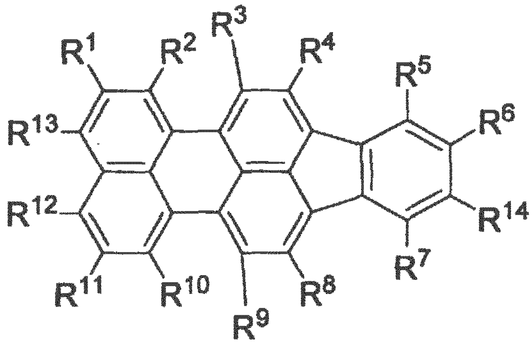

(B-1)

wherein $R^1$ to $R^{14}$ are independently an amino group represented by the following formula (B-2), a hydrogen atom, a halogen atom, a substituted or unsubstituted alkyl group having 1 to 40 carbon atoms, a substituted or unsubstituted alkenyl group having 2 to 40 carbon atoms, a substituted or unsubstituted alkynyl group having 2 to 40 carbon

atoms, a substituted or unsubstituted aryl group having 6 to 40 ring carbon atoms, a substituted or unsubstituted heteroaryl group having 5 to 40 ring atoms, a substituted or unsubstituted alkoxy group having 1 to 40 carbon atoms or a substituted or unsubstituted aryloxy group having 6 to 40 ring carbon atoms, at least one of $R^1$ to $R^{14}$ is an amino group represented by the following formula (B-2); and adjacent groups of $R^1$ to $R^{14}$ may be bonded to each other to form a ring:

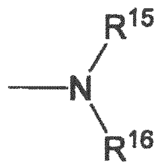

(B-2)

wherein $R_{15}$ and $R_{16}$ are independently a substituted or unsubstituted aryl group having 6 to 40 ring carbon atoms or a substituted or unsubstituted alkyl group having 1 to 40 carbon atoms.

**9.** The material for an organic thin film solar cell according to claim 8, wherein the indenoperylene derivative represented by the formula (B-1) is represented by the following formula (B-3):

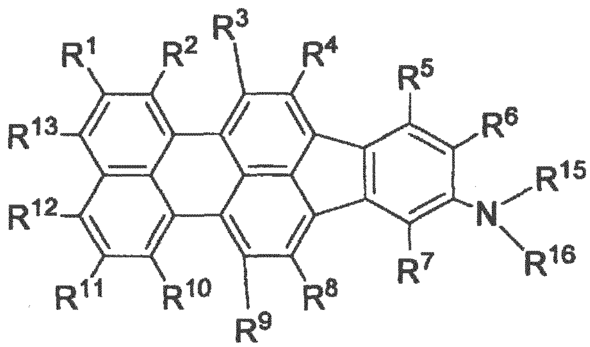

(B-3)

wherein $R^1$ to $R^{13}$ and $R^{15}$ and $R^{16}$ are the same as those in the formulas (B-1) and (B-2).

**10.** An organic thin film solar cell comprising the material for an organic thin film solar cell according to claim 8 or 9.

**11.** An organic thin film solar cell having at least a p layer, wherein the p layer comprises the material for an organic thin film solar cell according to claim 8 or 9.

**12.** An organic thin film solar cell comprising at least a p layer and an n layer, wherein the n layer comprises a fullerene derivative and the p layer comprises the material for an organic thin film solar cell according to claim 8 or 9.

**INTERNATIONAL SEARCH REPORT**

International application No.
PCT/JP2011/004232

A. CLASSIFICATION OF SUBJECT MATTER
*C07C211/61*(2006.01)i, *H01L51/42*(2006.01)i

According to International Patent Classification (IPC) or to both national classification and IPC

B. FIELDS SEARCHED

Minimum documentation searched (classification system followed by classification symbols)
C07C211/61, H01L51/42

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO 01/23497 A1 (Idemitsu Kosan Co., Ltd.), 05 April 2001 (05.04.2001), entire text & US 2006/0024523 A1 & EP 1138745 A1 | 1-12 |
| A | JP 2005-113071 A (Toyo Ink Manufacturing Co., Ltd.), 28 April 2005 (28.04.2005), entire text (Family: none) | 1-12 |
| A | JP 2007-230887 A (Idemitsu Kosan Co., Ltd.), 13 September 2007 (13.09.2007), entire text (Family: none) | 1-12 |

☒ Further documents are listed in the continuation of Box C.    ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 October, 2011 (14.10.11) | 25 October, 2011 (25.10.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No. PCT/JP2011/004232

C (Continuation). DOCUMENTS CONSIDERED TO BE RELEVANT

| Category* | Citation of document, with indication, where appropriate, of the relevant passages | Relevant to claim No. |
|---|---|---|
| A | JP 2003-272866 A (Petroleum Energy Center), 26 September 2003 (26.09.2003), entire text (Family: none) | 1-12 |
| A | WO 2007/099983 A1 (Idemitsu Kosan Co., Ltd.), 07 September 2007 (07.09.2007), entire text & US 2008/0007160 A1 & EP 1990843 A1 | 1-12 |
| A | JP 2008-135540 A (Sanyo Electric Co., Ltd.), 12 June 2008 (12.06.2008), entire text (Family: none) | 1-12 |
| A | JP 2009-132674 A (Idemitsu Kosan Co., Ltd.), 18 June 2009 (18.06.2009), entire text & WO 2009/057430 A1 | 1-12 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2008135540 A
- JP 2009132674 A
- JP 2008034764 A
- JP 2007335760 A
- WO 2001023497 A
- WO 201001352 A
- WO 2010013520 A